(19) Europäisches Patentamt
European Patent Office
Office européen des brevets

(11) Veröffentlichungsnummer: **0 312 086 B1**

(12) **EUROPÄISCHE PATENTSCHRIFT**

(45) Veröffentlichungstag der Patentschrift: **02.02.94**

(51) Int. Cl.$^5$: **C07H 15/04**, A61K 31/72

(21) Anmeldenummer: **88117115.1**

(22) Anmeldetag: **14.10.88**

# (54) Polyschwefelsäureester von Bis-aldonsäureamiden und deren Derivaten, Verfahren zu ihrer Herstellung und Arzneimittel.

(30) Priorität: **14.10.87 DE 3734815**

(43) Veröffentlichungstag der Anmeldung:
**19.04.89 Patentblatt 89/16**

(45) Bekanntmachung des Hinweises auf die Patenterteilung:
**02.02.94 Patentblatt 94/05**

(84) Benannte Vertragsstaaten:
**AT BE CH DE ES FR GB GR IT LI LU NL SE**

(56) Entgegenhaltungen:
**DE-A- 1 124 938**
**FR-A- 2 358 418**
**FR-A- 2 587 030**
**US-A- 2 752 334**

**DIE MAKROMOLEKULARE CHEMIE - RAPID COMMUNICATIONS, Band 5, Nr. 7, Juli 1984, Seiten 373-379, Basel, CH; G. ZIEGAST et al.: "Linear and star-shaped hybrid polymers, 2a"**

(73) Patentinhaber: **LUITPOLD PHARMA GmbH**
**Postfach 70 12 09**
**D-81312 München(DE)**

(72) Erfinder: **Meinetsberger, Eike, Dr. rer. nat.**
**Friedenheimerstrasse 145**
**D-8000 München 21(DE)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft Polyschwefelsäureester von Bis-aldonsäureamiden und deren Derivaten der allgemeinen Formel I,

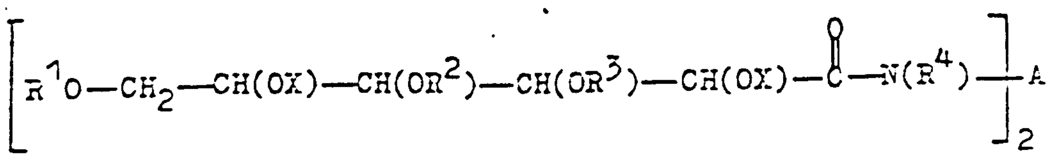

(I)

worin entweder
sämtliche Reste $R^1$, $R^2$ und $R^3$ unabhängig voneinander für X stehen, oder
zwei der Reste $R^1$, $R^2$ und $R^3$ für X, und der dritte für einen Rest der Formeln II - VII stehen,

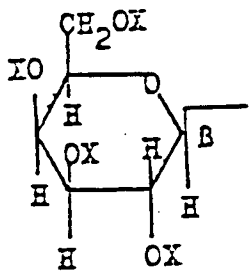

(II)

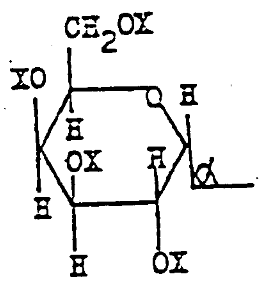

(III)

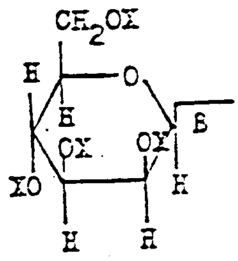

(IV)

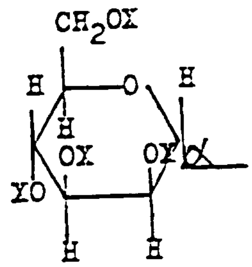

(V)

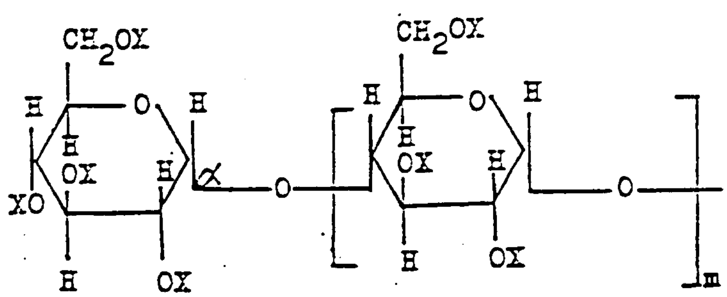

(VI)

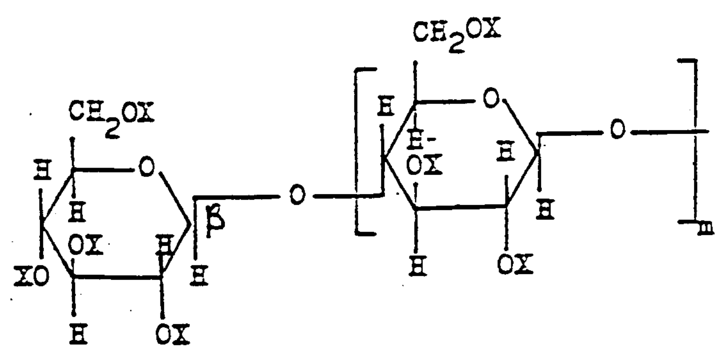

(VII)

X in den Formeln I bis VII gleichzeitig oder unabhängig voneinander ein Wasserstoffatom oder die Gruppe $-SO_3H$ bedeutet, wobei mindestens ein X für die Gruppe $-SO_3H$ steht,

m für 0,1,2,3,4,5 oder 6 steht,

A in Formel I für einen gegebenenfalls durch einen oder mehrere Reste $-CO_2R^5$ substituierten geradkettigen oder verzweigten, gesättigten Alkylenrest mit 2 bis 22

Kohlenstoffatomen steht, und dieser Alkylenrest gegebenenfalls durch bis zu 5 -O-, -S-, -S-S-, $-S(O)_n-$,

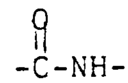

oder/und $-NR^6$-Gruppen oder Cycloalkylen- oder Arylenreste unterbrochen ist, oder A für eine einfache Bindung oder den Rest

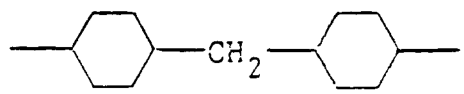

steht,

n 1 oder 2 ist,

$R^4$, $R^5$ und $R^6$ gleichzeitig oder unabhängig voneinander ein Wasserstoffatom oder einen $C_1$-$C_6$-Alkylrest bedeuten,

sowie deren Salze mit anorganischen oder organischen Basen.

Diese erfindungsgemäßen Verbindungen stellen Wirkstoffe mit wertvollen pharmakologischen Eigenschaften dar, wie weiter unten ausgeführt wird.

Für die im Zusammenhang mit der vorliegenden Erfindung und Beschreibung genannten verschiedenen Substituenten beziehungsweise Reste (in den verschiedenen angegebenen Formeln) gelten folgende Erläuterungen:

Die den vorliegenden Polyschwefelsäureestern zugrundeliegenden Aldonsäuren besitzen die allgemeine Formel VIII

$$R^1O—CH_2—CH(OH)—CH(OR^2)—CH(OR^3)—CH(OH)—CO_2H \quad (VIII)$$

worin $R^1$, $R^2$ und $R^3$ die angegebene Bedeutung besitzen. Diese Aldonsäuren können in der D-Form, der L-Form oder in Form ihrer Razemate, bevorzugt in ihrer in der Natur überwiegenden Form, vorliegen.

Beispiele für diese Aldonsäuren umfassen die Hexonsäuren Allonsäure, Altronsäure, Galaktonsäure, Gluconsäure, Gulonsäure, Idonsäure, Mannonsäure und Talonsäure, bevorzugt Galaktonsäure, Gluconsäure, Gulonsäure und Mannonsäure. Weitere Beispiele sind Derivate dieser Hexonsäuren, welche an den Sauerstoffatomen in 3-, 4-, oder 6-Stellung glycosidisch mit einem Rest $R^3$ oder $R^2$ oder $R^1$ der Formeln II bis VII, in denen X für Wasserstoff steht, verbunden sind. Die Bindung kann hier $\alpha$- oder $\beta$-glykosidisch sein. Bei den Resten II bis V handelt es sich um Galaktopyranosyl- und Mannopyranosylreste. Die Reste VI und VII sind Glucopyranosylreste (für den Fall, daß $m = 0$) und $\alpha(1\rightarrow4)$ bzw. $\beta(1\rightarrow4)$ -verknüpfte Oligoglucopyranosylreste (wenn $m = 1$ bis 6). Vorzugsweise steht in den Formeln VI und VII der Index m für 0 oder 1. Die mit der Aldonsäure verknüpften Saccharideinheiten liegen normalerweise in der D-Form vor. Beispiele für Hexonsäuren der allgemeinen Formel VIII, die mit Resten der Formeln II bis VII substituiert sind, sind Glucopyranosylgluconsäuren, Glucopyranosylmannonsäuren, Glucopyranosylgalaktonsäuren, Galaktopyranosylgluconsäuren, Mannopyranosylgluconsäuren, Mannopyranosylmannonsäuren und Oligoglucopyranosylgluconsäuren. Bevorzugt sind hier Lactobionsäure (4-O-$\beta$-D-Galaktopyranosylgluconsäure), Gentiobionsäure, Melibionsäure (6-O-$\alpha$-D-Galaktopyranosylgluconsäure), Mannobionsäure, Cellobionsäure (4-O-$\beta$-D-Glucopyranosylgluconsäure) und Maltobionsäure (4-O-$\alpha$-D-Glucopyranosylgluconsäure) sowie Maltotrionsäure und Cellotrionsäure.

Beispiele für salzbildende Basen sind Trialkylamine mit 1 bis 6 Kohlenstoffatomen im Alkylteil wie Trimethylamin, Triethylamin, Tripropylamin, Tributylamin, Tripentylamin und Trihexylamin. Bevorzugt sind Trimethylamin, Triethylamin und Tributylamin.

Beispiele für physiologisch verträgliche anorganische und organische Salze sind die Ammonium-, Lithium-, Natrium-, Kalium-, Magnesium-, Calcium-, Aluminiumsalze und die Salze mit Ethanolamin, Triethanolamin, Morpholin, Pyridin und Piperidin. Bevorzugt sind die Natrium-, Kalium-, Calcium-, Aluminium- und Ethanolaminsalze.

Beispiele für die Gruppe A darstellende geradkettige oder verzweigte, gesättigte Alkylenreste mit 2 bis 22 Kohlenstoffatomen sind Ethylen-, Tri-, Tetra-, Penta-, Hexa-, Hepta-, Octa-, Nona-, Deca-, Undeca-, Dodeca-, Tetradeca-, Hexadeca-, Octadeca-, Icosa- und Docosamethylen sowie Methylethylen, Methylpropylen, Methylbutylen, Methylpentylen und Dimethylethylen. Bevorzugt sind Ethylen-, Tri-, Tetra-, Hexa-, Nona-, Dodeca- und Docosamethylen sowie Methylethylen und Methylpentylen.

Beispiele für Arylenreste, durch die der Alkylenrest der Gruppe A unterbrochen sein kann, sind Phenylen, Naphthylen, Anthrylen, Phenanthrylen und Fluorenylen. Bevorzugt sind hierbei ortho-, meta- und para-Phenylenreste.

Beispiele für Cycloalkylenreste, durch die der Alkylenrest der Gruppe A unterbrochen sein kann, sind Cyclopentylen, Cyclohexylen, Cycloheptylen und Cyclooctylen, wobei hier 1,3- und 1,4-Cyclohexylen bevorzugt sind.

Vorzugsweise besitzt der geradkettige oder verzweigte, gesättigte Alkylenrest der Gruppe A 2 bis 12 Kohlenstoffatome. Ist der geradkettige oder verzweigte, gesättigte Alkylenrest der Gruppe A durch einen der genannten Reste oder Gruppen unterbrochen, handelt es sich vorzugsweise um 1 oder 2 derartige Reste bzw. Gruppen.

Spezielle Beispiele für definitionsgemäße, die Gruppe A darstellende Alkylenreste sind die sich von folgenden $\alpha,\omega$-Diaminen ableitenden Gruppen:

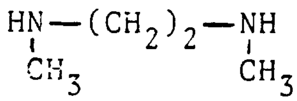

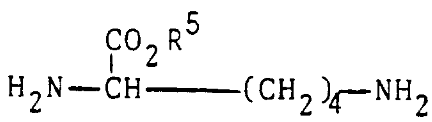

Enantiomere des Lysins ($R^5$ = H) und seiner Ester ($R^5$ = $C_1$-$C_6$-Alkyl)

<u>mit S-Atomen:</u>

$R^5O_2C$-HC($NH_2$)-$CH_2$S-$CH_2$-($NH_2$)CH-$CO_2R^5$

Diastereomere des Lanthionins ($R^5$ = H)
und Ester ($R^5$ = $C_1$-$C_6$-Alkyl)

$R^5O_2C$-HC($NH_2$)-$(CH_2)_x$-S-S-$(CH_2)_x$-($NH_2$)CH-$CO_2R^5$

Diastereomere des Cystins (x = 1, $R^5$ = H)
und Ester ($R^5$ = $C_1$-$C_6$-Alkyl)
Diastereomere des Homocystins (x = 2, $R^5$ = H)
und Ester ($R^5$ = $C_1$-$C_6$-Alkyl)

$HO_2C$-CH($NH_2$)-$(CH_2)_2$-S-$CH_2$-CH($NH_2$)-$CO_2H$

Diastereomere des Cystathionins

<u>mit NH-Gruppen:</u>

| | |
|---|---|
| $H_2N(-CH_2-CH_2-NH)_x-CH_2-CH_2-NH_2$ | x = 1 Diethylentriamin |
| | x = 2 Triethylentetramin |
| | x = 3 Tetraethylenpentamin |
| $H_2N-(CH_2)_2-NH-(CH_2)_3-NH-(CH_2)_2-NH_2$ | 1,9-Diamino-3,7-diazanonan |
| $H_2N-(CH_2)_3-NH-(CH_2)_2-NH-(CH_2)_3-NH_2$ | 1,10-Diamino-4,7-diazadecan |
| $H_2N-(CH_2)_6-NH-(CH_2)_6-NH_2$ | Bis-(6-aminohexyl)amin |
| $H_2N-(CH_2)_3-NH-(CH_2)_4-NH-(CH_3)_3-NH_2$ | Spermin |
| $H_2N-(CH_2)_4-NH-(CH_2)_3-NH_2$ | Spermidin |
| $H_2N-(CH_2)_3-NH-(CH_2)_3-NH-(CH_2)_3-NH_2$ | 1,11-Diamino-4,8-diazaundecan |

mit O-Atomen:

$H_2N-(CH_2)_2-O-(CH_2)_2-NH_2$ Bis-(2-aminoethyl)ether

Vorzugsweise kann die Gruppe A für folgende Reste stehen:

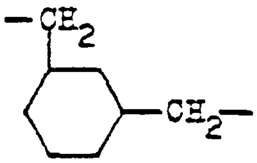 ;

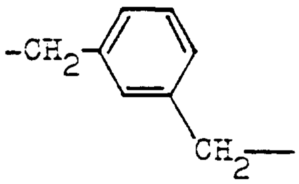 ; 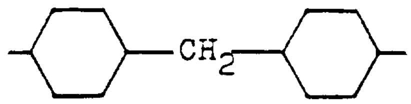 ;

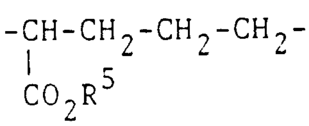

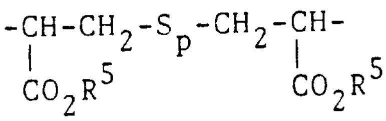 mit p = 1 oder 2

$-(CH_2)_2-S-S-(CH_2)_2-$

$-(CH_2)_3-O-(CH_2)_4-O-(CH_2)_3-$

$-(CH_2)_3-NH-(CH_2)_3-$

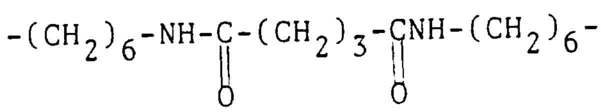

Beispiele für $C_1$ - $C_6$-Alkylreste der Gruppen $R^4$, $R^5$ und $R^6$ sind Methyl, Ethyl, n-Propyl, n-Butyl, n-Pentyl, n-Hexyl, Isopropyl, Isobutyl, tert.-Butyl, Neopentyl, wobei Methyl, Ethyl, n-Propyl, Isopropyl, tert.-Butyl und n-Butyl bevorzugt sind.

Die Erfindung betrifft auch ein Verfahren zur Herstellung von Polyschwefelsäureestern von Bis-aldonsäureamiden der allgemeinen Formel I, das dadurch gekennzeichnet ist, daß man Bis-Aldonsäureamide der allgemeinen Formel IX

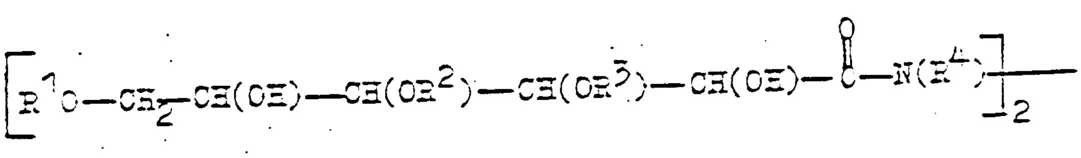 (IX)

worin $R^1$, $R^2$, $R^3$, $R^4$ und A die angegebenen Bedeutungen haben, wobei jedoch X in den Formeln II bis VII für Wasserstoff steht, in einem aprotischen Lösungsmittel mit einem Sulfatierungsmittel umsetzt und die so erhaltenen Produkte mit einer anorganischen oder organischen Base in die entsprechenden Salze überführt.

Man erhält die Verbindungen der allgemeinen Formel IX in Analogie zu literaturbekannten Verfahren (z.B.: F. Scholnick, P.E. Pfeffer, J. Dairy Sci. 63 (3), 471 (1980); W.N. Emmerling, B. Pfannemüller, Starch 33 (6), 202 (1981)). Zur Herstellung der Bis-aldonsäureamide der allgemeinen Formel IX läßt man Lactone der Aldonsäuren der allgemeinen Formel VIII mit einer Diaminoverbindung $R^4HN\text{-}A\text{-}NHR^4$ in einem Lösungsmittel reagieren. Die Lactone können dabei sowohl in der 1,4-Lactonform der allgemeinen Formel X als auch in der 1,5-Lactonform der allgemeinen Formel XI eingesetzt werden.

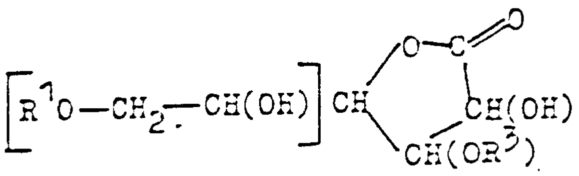

(X)

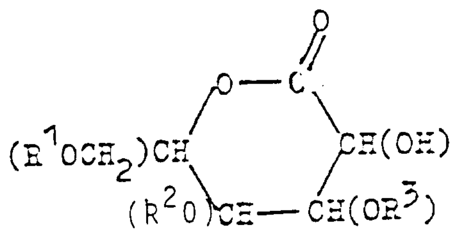

(XI)

Man erhält die Verbindungen der allgemeinen Formeln X und XI durch Wasserabspaltung aus den Aldonsäuren VIII. Die Aldonsäuren können nach literaturbekannten Verfahren (z.B. W.N. Emmerling, B. Pfannemüller, Starch 33 (6), 202 (1981); R. Schaffer, H. S. Isbell, J. Am. Chem. Soc. 81, 2178 (1959), H. W. Diehl et al., Carbohydrate Research 38, 364 (1974)) durch elektrochemische oder Hypohalogenit-Oxidation aus den entsprechenden Aldosen gewonnen werden. Für die Herstellung der Bis-aldonsäureamide der allgemeinen Formel IX setzt man pro Mol Diaminoverbindung 2 Mol Aldonsäurelacton ein. Geeignete Lösungsmittel für die Umsetzung sind Methanol, Ethanol, Ethylenglykol, Dimethylsulfoxid , Dimethylformamid oder N-Methylpyrrolidon. Bevorzugt wird Dimethylformamid. Die Reaktionszeiten betragen mehrere Stunden bis Tage, bevorzugt zwischen 5 und 8 h. Die Reaktionstemperaturen liegen zwischen Raumtemperatur und den Siedetemperaturen der jeweiligen Lösungsmittel, bevorzugt zwischen 40 °C und 80 °C. Die Aldonsäureamide kristallisieren entweder aus der Reaktionslösung aus oder man kann sie durch Zusatz eines organischen Lösungsmittels ausfällen. Geeignet hierfür sind Methanol, Ethanol, Isopropanol oder Aceton, vorzugsweise Isopropanol.

Die Bis-aldonsäureamide der allgemeinen Formel IX sind Gegenstand der DE-A-3734853 der gleichen Anmelderin vom gleichen Anmeldetag; Titel "Bis-aldonsäureamide und Verfahren zur ihrer Herstellung".

Für die Herstellung der vorliegenden Verbindungen der allgemeinen Formel I löst oder suspendiert man die Bis-aldonsäureamide der Formel IX in einem aprotischen Lösungsmittel. Geeignet sind Pyridin, Dimethylformamid, Dimethylacetamid, N-Methylpyrrolidon etc., bevorzugt wird Dimethylformamid.

Man erwärmt das Gemisch auf Temperaturen zwischen 20 °C und 100 °C, bevorzugt zwischen 30 °C und 70 °C, und setzt ein Sulfatierungsmittel zu. Beispiele hierfür sind Chlorsulfonsäure, Schwefel-trioxid, Oleum, Ether- oder Amin-Schwefeltrioxid-Komplexe. Bevorzugt werden die Schwefeltrioxid-Komplexe mit Trimethylamin, Triethylamin und Pyridin eingesetzt.

Um Polyschwefelsäureester von Bis-aldonsäureamiden der allgemeinen Formel I zu erhalten, worin jedes X für die Gruppe $-SO_3H$ steht, setzt man für jede veresterungsfähige Hydroxylgruppe 1 bis 2 Äquivalente Sulfatierungsmittel ein, bevorzugt 1,4 - 1,7 Äquivalente. Verbindungen, bei denen nicht jedes X für die Gruppe $-SO_3H$ steht, erhält man, wenn man die Sulfatierungsmittel im Unterschuß einsetzt. Je nachdem, wie hoch der Schwefelgehalt des Produktes sein soll, wählt man die Menge des Sulfatierungsmittels. Bevorzugt sind Mengen zwischen 0,5 und 1 Äquivalent Sulfatierungsmittel pro veresterungsfähiger Hydroxylgruppe. Man rührt das Reaktionsgemisch 1 bis 24 h bei Temperaturen zwischen 20 °C und 100 °C. Aus den so gewonnenen Polysulfatverbindungen der allgemeinen Formel I, worin X für $-SO_3H$ steht, die auch als Salz des Amins des eingesetzten Amin-Schwefeltrioxid-Komplexes vorliegen können, erhält man

die entsprechenden Salze anorganischer Basen durch Zugabe dieser Basen oder deren anorganischer Salze. Bevorzugt werden hier die Hydroxide und Acetate der Alkali- und Erdalkalimetalle eingesetzt. Salze physiologisch unbedenklicher organischer Basen erhält man entweder durch Verwendung eines Schwefeltrioxid-Komplexes dieser Basen bei der Sulfatierungsreaktion oder durch Behandeln der Erdalkali- oder Alkalisalze mit einem Kationenaustauscher in der sauren Form und anschließender Neutralisation der sauren Schwefelsäurehalbester mit der organischen Base. Die Isolierung der erfindungsgemäßen Verbindungen erfolgt im allgemeinen durch Fällung aus den Reaktionslösungen oder aus wässrigen Lösungen mit organischen Lösungsmitteln. Geeignete organische Lösungsmittel sind Methanol, Ethanol, Isopropanol oder Aceton, vorzugsweise Methanol.

Die Verbindungen lassen sich durch wiederholtes Umfällen aus wässrigen Lösungen mit den oben genannten Lösungsmitteln und durch Behandeln mit Aktivkohle oder Wasserstoffperoxid reinigen. Im allgemeinen handelt es sich bei den so erhaltenen Produkten um Gemische mit einem mehr oder weniger hohen Gehalt an Verbindungen, deren sämtliche Hydroxylgruppen mit Sulfatresten verestert sind und an Verbindungen, deren Hydroxylgruppen nur teilweise sulfatiert sind, je nach Menge des eingesetzten Sulfatierungsmittels. Die erhaltenen Gemische können gegebenenfalls durch verschiedene Trennverfahren in Produkte mit einem einheitlichen Gehalt an Sulfatresten aufgetrennt werden. Die Auftrennung kann nach unterschiedlichen physikalischen Trennverfahren geschehen, wie zum Beispiel fraktionierter Fällung, Gel-, Ionenaustausch- oder Affinitätschromatographie, HPLC oder elektrophoretischen Verfahren. Bevorzugt wird die fraktionierte Fällung. Zum Beispiel stellt man eine wässrige Lösung des Produktgemisches her und versetzt mit der ein- bis vierfachen Menge eines organischen Lösungsmittels. Geeignet sind mit Wasser mischbare Lösungsmittel wie Methanol, Ethanol, Isopropanol, Aceton oder Tetrahydrofuran, bevorzugt wird Methanol. Den so erhaltenen Niederschlag läßt man absetzen. Im allgemeinen sind nun die Verbindungen mit höherem Sulfatgehalt in der Fällung angereichert, die mit niedrigerem Sulfatgehalt in der überstehenden Lösung. Durch wiederholtes fraktioniertes Fällen der Niederschläge oder Überstände erhält man Verbindungen mit einer definierten Anzahl von Sulfatgruppen.

In einer bevorzugten Ausführungsform des erfindungsgemäßen Verfahrens werden die Verbindungen der Formel I aus den Aldonsäuren der Formel VIII ohne Isolierung der nötigen Zwischenstufen erzeugt.

Hierbei stellt man aus den käuflichen oder nach bekannten Literaturverfahren (zum Beispiel W. N. Emmerling, B. Pfannemüller, Starch 33 (6), 202 (1981); R. Schaffer, H. S. Isbell, J. Am. Chem. Soc. 81, 2178 (1959), H. W. Diehl et al., Carbohydrate Research 38, 364 (1974)) synthetisierten Alkali- oder Erdalkalisalzen der Aldonsäuren VIII mittels eines Kationenaustauschers eine wässrige Lösung der freien Aldonsäuren VIII her und engt diese weitgehend ein. Die den Aldonsäuren VIII entsprechenden Lactone werden nun in situ durch Wasserabspaltung erzeugt. Zu diesem Zweck löst man den Rückstand, der im allgemeinen ein wasserhaltiges Gemisch aus Aldonsäure und Lacton darstellt, in einem hochsiedenden Lösungsmittel. Beispiele für hochsiedende Lösungsmittel sind Dimethylsulfoxid, Dimethylformamid, N-Methylpyrrolidon, Dimethoxymethylether etc., bevorzugt wird Dimethylformamid. Nun setzt man ein zweites, niedrigsiedendes Lösungsmittel zu, welches ein Azeotrop mit Wasser bilden kann. Geeignete Lösungsmittel sind zum Beispiel n-Pentan, n-Hexan, Cyclohexan, Benzol etc., bevorzugt ist n-Hexan. Am Wasserabscheider wird nun aus den Aldonsäuren quantitativ Wasser abgespalten. Dann destilliert man das niedrigsiedende Schleppmittel ab und setzt das im zurückbleibenden, hochsiedenden Lösungsmittel befindliche Lacton, ohne Isolierung desselben, mit der Diaminoverbindung um. Die Reaktionstemperaturen liegen dabei zwischen 20 °C und 120 °C, bevorzugt zwischen 50 °C und 80 °C. Man rührt über Zeitspannen von 3 bis 24 Stunden, bevorzugt 3 bis 8 Stunden. Ohne Isolierung des entstandenen Bis-aldonsäureamids, wird dieses im selben Reaktionsgefäß mit einem Sulfatierungsmittel zur Reaktion gebracht. Reaktionsbedingungen sowie Isolierung und Reinigung der Produkte sind im vorher genannten Verfahren beschrieben.

Die Verbindungen der allgemeinen Formel I stellen pharmakologisch wertvolle Substanzen dar. Sie besitzen antithrombotische und antientzündliche Eigenschaften. Die erfindungsgemäßen Verbindungen sind deshalb zur Behandlung von Erkrankungen des rheumatischen Formenkreises sowie zur Prophylaxe und Therapie von venösen und arteriellen Thrombosen geeignet. Gegenstand der Erfindung ist daher auch ein Arzneimittel für die Verwendung bei Mensch und Tier. Bevorzugt ist der Gebrauch beim Menschen.

Besonders überraschend ist die antithrombotische Wirksamkeit der erfindungsgemäßen Verbindungen.

Bislang wurde zur Thromboseprophylaxe in erster Linie Heparin verwendet. Heparin ist ein Mucopolysaccharid, das aus tierischem Gewebe, insbesondere Schweinedarm isoliert wird (Thomas, D.P. (1981) in Clinics in Haematology, Vol. 10, S. 443 - 458, Saunders Comp. Ltd., 1981). Neben Heparin besitzen auch andere natürlich vorkommende Mucopolysaccharide wie Dermatansulfat oder Heparansulfat antithrombotische Eigenschaften (Rosenberg, R.D., Rosenberg, J.S. (1984), J. Clin. Invest. 74, 1 - 6; Sie, P., Ofosu, F., Fernandez, F., Buchanan, M.R., Petitou M., Boneu, B.(1986) Br. J. Haematol. 64, 707 - 714). Es ist auch ein semisynthetisches Chondroitinpolysulfat z.B. aus der DE-PS 31 18 588 bekannt, das unter anderem

antithrombotische Eigenschaften aufweist. Nur Heparin wird jedoch therapeutisch verwendet. In jüngster Zeit sind einige niedermolekulare Heparine zur klinischen Anwendung entwickelt worden. Hierbei handelt es sich um Substanzen, die durch verschiedene chemische oder enzymatische Depolymerisationsverfahren aus Heparin gewonnen werden (Thomas, D.P., Merton, R.E. (1982) Thrombos. Res. 28, 343 - 350; Walenga, J.M., Fareed, J., Petitou, M., Samana, M., Lormeau, J.C., Choay, J. (1986) Thrombos. Res. 43, 243 - 248; Koller, M., Schoh, U., Buchmann, P. Largiadèr, F., von Felten, A., Frick, P.G. (1986) Thrombos. Haemostas. 56, 243 - 246).

Ein Nachteil der Heparine und niedermolekularen Heparine besteht darin, daß sie natürlichen Ursprungs (z.B. von Tieren stammend) sind. Aus dem tierischen Gewebe stammend sind daher geringe Mengen von Antigen vorhanden, das zu anaphylaktoiden Reaktionen wie Thrombozytenabfall, Thrombose und Embolie führen kann. Diese Nebenwirkungen sind zwar relativ selten, doch schwerwiegend und klinisch schwer beherrschbar.

Demgegenüber sind die erfindungsgemäßen Verbindungen vollsynthetisch und daher frei von tierischen Antigen.

Eine andere wesentlich häufigere Komplikation bei der Thromboseprophylaxe mit Heparin und niedermolekularem Heparin ist das Auftreten von Blutungen. Substanzen, die bei gleicher antithrombotischer Wirkung weniger Blutungen verursachen, können daher einen wesentlichen therapeutischen Fortschritt darstellen.

Zur Prüfung der erfindungsgemäßen Verbindungen auf antithrombotische Wirksamkeit und antientzündliche Wirksamkeit wurden mehrere pharmakologische Tests verwendet.

1. Beschleunigung der Fibrinolyse

Die Entstehung eines klinisch manifesten (größeren) Thrombus kann auf verschiedene Weise verhindert werden. Zum einen können die Faktoren, die an der Bildung des primären Thrombus beteiligt sind, wie Blutplättchenaggregation oder Blutgerinnungssystem, ausgeschaltet werden. Andererseits kann durch Verstärkung der körpereigenen Fibrinolyse die Auflösung des Primärthrombus beschleunigt werden. Eine Verstärkung der körpereigenen Fibrinolyse kann auch dazu dienen, bereits klinisch manifeste Thromben wieder aufzulösen. Der fibrinolytischen Wirkung einer Substanz kommt somit bei Prophylaxe und Therapie von Thrombosen eine große Bedeutung zu. Die fibrinolytische Wirkung der erfindungsmäßigen Substanzen wurde im nachstehenden Test bestimmt. Der Test ist eine leichte Modifikation einer literaturbekannten Methode zur Bestimmung der fibrinolytischen Wirkung (Kluft, C. (1979) Thromb. Haemostas. 41, 365 - 383).

Fibrinolyse-Test

Die Fibrinolyse wurde mittels plasminogenhaltiger Fibrinplatten bestimmt.

Petrischalen, ∅ 9 cm, wurden auf 40 °C vorgewärmt. Unter Schwenken der Schale wurden 3 ml einer 2 % Lösung von Fibrinogen (Behringwerke, Marburg) in Wasser, 1 ml einer Plasminogenlösung, 2 CTA/ml (Behringwerke, Marburg), 3 ml einer 1 % Agaroselösung (Serva, Heidelberg) in 50 mM TRIS/HCl, pH 7,8 und 1 ml einer Lösung von 15 U/ml Thrombin (Behringwerke, Marburg) in Wasser einpipettiert. Nach Abkühlen der Platte wurden Löcher von 5 mm Durchmesser ausgestanzt.

0,1 ml Plasma (Humanes Standardplasma, Behringwerke, Marburg), 0,8 ml Wasser und 0,1 ml Lösung der Testsubstanz in verschiedenen Konzentrationen wurden 10 Minuten bei 37 °C inkubiert. Nach Zusatz von 0,9 ml 0,025 % Essigsäure wurde 5 Minuten bei 4 °C inkubiert und anschließend bei 2000 g 5 Minuten zentrifugiert. Der Niederschlag wurde in 0,1 ml Puffer (20 mM TRIS/HCl, 100 mM NaCl, 2,7 mM EDTA, pH 7,8) aufgenommen.

0,02 ml dieser Lösung und 0,005 ml 14,3 mM Flufenaminsäurelösung (Sigma, Taufkirchen) in Puffer werden in die Auftragelöcher der Fibrinplatten pipettiert. Nach 24 Stunden bei 37 °C wurden die Lysehöfe planimetriert. Die Fläche der Lysehöfe abzüglich der Lysefläche ohne Substanzzusatz diente als Maß für die fibrinolysesteigernde Aktivität.

Ergebnis: Das Ergebnis ist in der nachstehenden Tabelle dargestellt.

| Substanz aus Beispiel | Fibrinolyse bei 2 µg/ml ($mm^2$) |
|---|---|
| 23 | 21,6 |
| 20 | 19,2 |
| 24 | 17,8 |
| Heparin | 14,8 |

2. Hemmung der Blutgerinnung

Die Hemmung der Blutgerinnung kann auf einfache Weise mittels der aktivierten partiellen Thromboplastinzeit aPTT gemessen werden. Sie gibt einen Aufschluß über die generelle Hemmung des aktivierten Gerinnungssystems. Um die Inzidenz von Blutungen zu verringern, ist hier eine möglichst geringe Hemmung der Blutgerinnung erwünscht.

Gerinnungs-Test. aPTT

0,1 ml Plasma und 0,1 ml Pathromtin[R] (Behringwerke, Marburg) werden 2 Minuten bei 37 °C inkubiert und mit 0,1 ml 25 mM $CaCl_2$-Lösung versetzt. Man mißt die Gerinnungszeit in einem Schnitger & Gross Coagulometer. Pro Konzentration wird das Mittel aus 5 Einzelbestimmungen gebildet. Als Standard dient ein Heparin der Firma Pharmindustrie mit 175 E/mg.

Die Verlängerung der Gerinnungszeit wird über eine Eichkurve in Heparin Einheiten umgewandelt. Da die Eichkurven nicht parallel sind, wird die Verlängerung der Gerinnungszeit auf 150 % des Ausgangswerts als Bezugspunkt gewählt.

Ergebnis: Die folgende Tabelle zeigt die Heparin Einheiten erfindungsgemäßer Verbindungen.

| Substanz aus Beispiel | E/mg |
|---|---|
| 23 | 30,9 |
| 25 | 5,85 |
| 20 | 42,1 |
| 24 | 14,1 |
| 31 | 10,2 |
| 34 | 15,1 |
| Heparin | 175 |
| Fragmin (niedermol. Heparin) | 64,5 |

3. Beeinflussung der Blutungszeit

Ratten wurde ein ca. 2 mm langes Stück des Schwanzes gekappt. Die Zeit bis zum Ende der Blutung wurde gemessen. Den Tieren wurde 5 Minuten vor Auslösen der Blutung die Prüfsubstanz in verschiedenen Konzentrationen oder physiologische Kochsalzlösung (Kontrolle) verabreicht. Es wurden Gruppen à 20 Tiere behandelt. Die Verlängerung der Blutungszeit gegenüber der Kontrolle wurde in Prozent ausgedrückt.

| Substanz aus Beispiel | Verlängerung der Blutungszeit (%) | | |
|---|---|---|---|
| | Dosis (mg/kg) | | |
| | 0,25 | 0,5 | 2 |
| 20 | - | 5,1 | 35,1 |
| Heparin | 55,4 | 120 | |

4. Antientzündliche Wirkung

Bei entzündlichen Prozessen werden aus den Phagozyten (polymorphkernige Leukozyten und Makrophagen) große Mengen reaktiver Sauerstoffspezies freigesetzt, darunter Superoxidradikale und Hydroxylradikale. Diese Radikale sind an der Gewebszerstörung beteiligt. Substanzen, die die Radikalbildung in Leukozyten hemmen, sind deshalb von großem therapeutischen Interesse bei der Bekämpfung von Entzündungen (Flohé, L., Giertz, H., Beckmann, R. (1985) in The Pharmacology of inflammation, Vol 5 (Bonta, I. et al. eds.) S. 255 - 281, Elsevier, Amsterdam-New York.

Die Radikalbildung von Leukozyten kann durch Messung der Luminol-verstärkten Chemilumineszenz in Vollblut gemessen werden (Peter, M. et al. (1985) in Chi. Forum 85 (Stelzner, F., eds), S. 81 - 84, Springer Verlag, Berlin).

Test auf antientzündliche Wirkung

Testsubstanzen werden in PBS gelöst und Verdünnungsreihen in PBS angesetzt. In Küvetten, die zu einem 1251 Luminometer von LKB passen, werden 600 µl PBS, 100 µl Lösung der Testsubstanz, 100 µl Zymosansuspension (100 mg/ml) und 100 µl Kaninchencitratblut pipettiert. Die Messung wird durch Zusatz von 100 µl $10^{-3}$ M Luminol-Lösung gestartet. Die Chemilumineszenz wird über 90 Minuten alle 5 Minuten in jedem Ansatz gemessen. Die maximale Chemilumineszenz wird ermittelt. Als Referenzwert dient ein Ansatz ohne Testsubstanz. Als Leerwert dient ein Ansatz ohne Zymosan-Zusatz. Es werden pro Substanz drei unabhängige Bestimmungen durchgeführt. Die Berechnung der restlichen Chemilumineszenz erfolgt nach der Formel:

$$\frac{CL_{max}\,(Subst) - CL_{max}\,(Leer)}{CL_{max}\,(Ref) - CL_{max}\,(Leer)} \times 100$$

Aus einer semilogarithmischen Auftragung restlicher Chemilumineszenz über log Konzentration ergeben sich Konzentrationen der 50 % CL Hemmung ($IC_{50}$-Werte).

Ergebnis:

Die Hemmung der Luminol-verstärkten CL durch erfindungsmäßige Verbindungen ist in der nachstehenden Tabelle gezeigt.

| Substanz aus Beispiel | $IC_{50}$ (µg/ml) |
|---|---|
| 23 | 225 |
| 25 | 155 |
| 20 | 540 |
| 24 | 220 |
| 31 | 170 |
| Indomethacin | 240 |

Gegenstand der vorliegenden Erfindung sind auch pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder, Sprays und Aerosole genannt.

Tabletten, Dragees, Kapseln, Pillen und Granulate können den oder die Wirkstoffe neben den üblichen Trägerstoffen enthalten, wie a) Füll- und Streckmittel, zum Beispiel Stärken, Milchzucker, Rohrzucker, Glucose, Mannit und Kieselsäure, b) Bindemittel, zum Beispiel Carboxymethylcellulose, Alginate, Gelatine, Polyvinylpyrrolidon, c) Feuchthaltemittel, zum Beispiel Glycerin, d) Sprengmittel, zum Beispiel Agar-Agar, Calciumcarbonat und Natriumbicarbonat, e) Lösungsverzögerer, zum Beispiel Paraffin und f) Resorptionsbeschleuniger, g) Netzmittel, zum Beispiel Cetylalkohol, Glycerinmonostearat, h) Adsorptionsmittel, zum Beispiel Kaolin und Bentonit und i) Gleitmittel, zum Beispiel Talkum, Calcium- und Magnesiumstearat und feste Polyethylenglycole oder Gemische der unter a) bis i) aufgeführten Stoffe.

Die Tabletten, Dragees, Kapseln, Pillen und Granulate können mit den üblichen gegebenenfalls Opalisierungsmittel enthaltenden Überzügen, zum Beispiel Zucker, Überzugslacke, versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen zum Beispiel Polymersubstanzen und Wachse verwendet werden können.

Der oder die Wirkstoffe können gegebenenfalls mit einem oder mehreren der eben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel $C_{14}$-Alkohol mit $C_{16}$-Fettsäure oder Gemische dieser Stoffe).

Salben, Pasten, Cremes und Gele können neben dem oder den Wirkstoffen die üblichen Trägerstoffe enthalten, zum Beispiel tierische und pflanzliche Fette und deren Derivate, Wachse, Paraffine, Emulgatoren, Stärke, Traganth, Cellulosederivate, Polyacrylate, Polyethylenglycole, Silicone, Bentonite, Talkum, Kieselsäure und Zinkoxid oder Gemische dieser Stoffe.

Sprays und Puder können neben dem oder den Wirkstoffen die üblichen Trägerstoffe enthalten, zum Beispiel Milchzucker, Talkum, Kieselsäure, Aluminiumhydroxid, Calciumsilikat und Polyamidpulver oder Gemische dieser Stoffe. Sprays können zusätzlich die üblichen Treibmittel, zum Beispiel Chlorfluorkohlenwasserstoff enthalten.

Lösungen und Emulsionen können neben dem oder den Wirkstoffen die üblichen Trägerstoffe wie Lösungsmittel, Lösungsvermittler und Emulgatoren, zum Beispiel Wasser, Ethylalkohol, Isopropylalkohol, Ethylcarbonat, Ethylacetat, Benzylalkohol, Benzylbenzoat, Propylenglycol, 1,3-Butylenglycol, Öle, insbesondere Baumwollsaatöl, Erdnußöl, Cashewnußöl, Maiskeimöl, Olivenöl, Ricinusöl und Sesamöl, Glycerin, Glycerinformal, Polyethylenglycole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, zum Beispiel Ethylalkohol, Propylenglycol, Suspendiermittel, zum Beispiel ethoxylierte Isosteaylalkohole, Polyoxyethylensorbit- und Sorbitanester, mikrokristalline Cellulose, Aluminiummetahydroxid, Bentonit, Agar-Agar oder Traganth oder Gemische dieser Stoffe enthalten.

Die genannten Formulierungsformen können auch Färbemittel, Konservierungsstoffe sowie geruchs- und geschmacksverbessernde Zusätze, zum Beispiel Pfefferminzöl und Eucalyptusöl und Süßmittel, zum Beispiel Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Masse-% der Gesamtmischung vorhanden sein.

Die oben aufgeführten pharmazeutischen Zubereitungen können außer den erfindungsmäßigen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, zum Beispiel durch Mischen der Wirkstoffe mit den Trägerstoffen.

Zur vorliegenden Erfindung gehört auch die Verwendung der erfindungsgemäßen Wirkstoffe sowie der pharmazeutischen Zubereitungen, die einen oder mehrere Wirkstoffe enthalten, in der Human- und Veterinärmedizin zur Verhütung, Besserung und/oder Heilung der oben angeführten Krankheiten.

Die Wirkstoffe oder die pharmazeutischen Zubereitungen können lokal, oral, parenteral, rektal und/oder als Aerosol vorzugsweise parenteral appliziert werden.

Im allgemeinen hat es sich als vorteilhaft erwiesen, den oder die erfindungsgemäßen Wirkstoffe in Gesamtmengen von etwa 0,1 bis etwa 100, vorzugsweise 0,5 bis 50mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objektes, der Art und der Schwere der

Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Wirkstoffmenge auszukommen, während in anderen Fällen die oben angeführte Menge Wirkstoff überschritten werden muß.

Die nachfolgenden Beispiele erläutern die Erfindung näher, sollen ihren Umfang in keiner Weise beschränken.

Beispiel 1

N,N'-1,3-Propandiylbis-D-gluconamid

Man löst 7,13 g D(+)-Gluconsäure-1,5-lacton in 40 ml aminfreiem Dimethylformamid und versetzt mit 1,67 ml 1,3-Diaminopropan. Dann erwärmt man auf 60 °C und rührt 5 Stunden. Der entstandene Niederschlag wird abfiltriert, mit Methanol gewaschen und getrocknet. Man erhält 7,96 g eines weißen Pulvers.

| | |
|---|---|
| Schmelzpunkt: | 165 - 173 °C |
| IR (KBr): | ν = 3540, 2960, 2915, 2890, 1660, 1537, 1100, 1040 $cm^{-1}$ |
| $^1$H-NMR ($D_2O$): | δ 1,76 (dt, 2H, 6,5Hz); 3,30 (t, 4H, 6,5Hz); 3,4 - 4,0 (m, 8H); 4,09 (m, 2H); 4,30 (d, 2H, 3Hz); 4,70 ($H_2O$, i.St.) |

Beispiel 2

N,N'-1,12-Dodecandiylbis-D-gluconamid

Man suspendiert 7,1 g D-Gluconsäure-1,5-lacton in 90 ml Dimethylformamid, versetzt mit 4,0 g 1,12-Diaminododecan und rührt das Gemisch 5 Stunden bei 60 °C. Nach Abkühlen rührt man das Gemisch in 0,3 l Methanol ein, sammelt den Feststoff und wäscht ihn mit Methanol. Nun suspendiert man den Feststoff in 1 N HCl, rührt eine Stunde bei Raumtemperatur, sammelt den Feststoff erneut und wäscht ihn mit Wasser, Aceton und schließlich mit Diethylether. Man erhält 9,9 g eines weißen Pulvers.

| | |
|---|---|
| Schmelzpunkt: | 192 - 195 °C |
| IR (KBr): | ν = 2920, 2850, 1630, 1550, 1085, 1027 $cm^{-1}$ |
| $^1$H-NMR (DMSO - $d_6$): | δ 0,7 - 1,8 (m, 20H); 3,06 (m, 4H); 3,25 - 3,75 (m, 8H); 3,75 - 4,2 (m, 4H); 4,40 (S, 10H); 7,51 (t, 2H, 5,5Hz); i. St.: Tetramethylsilan |

Beispiel 3

N,N'-1,3-Propandiylbis [4-O-β-D-galactopyranosyl-D-gluconamid]

Man löst 395,4 g Calciumlactobionat in 1,2 l Wasser und behandelt die Lösung 1 Stunde mit 0,7 l Lewatit S 100 ($H^+$ Form) im Batch-Verfahren. Man saugt ab und wäscht den Austauscher mit 2 x 1 l Wasser. Die vereinigten Eluate engt man im Vakuum weitgehend ein. Nun löst man den glasartigen Rückstand in 800 ml aminfreiem Dimethylformamid, setzt 800 ml n-Hexan zu und erhitzt unter kräftigem Rühren am Wasserabscheider zum Sieden. Nach beendeter Wasserabscheidung destilliert man das n-Hexan ab, versetzt mit 43 ml 1,3-Diaminopropan und rührt 7 Stunden bei 63 °C. Nun rührt man das Gemisch in 5 l Isopropanol ein, sammelt den Feststoff und wäscht mit 1 l Isopropanol. Nach Trocknung erhält man 350 g eines weißen Feststoffes. Zur Reinigung löst man diesen in 2 l Wasser. Die Lösung behandelt man 1 Stunde mit 100 ml Lewatit S 100 ($H^+$-Form), dann mit 100 ml Amberlyst A 21 ($OH^-$-Form). Nach Gefriertrocknung erhält man die Titelverbindung in reiner Form.

| | |
|---|---|
| Schmelzpunkt: | 125 - 132 °C |
| IR (KBr): | ν = 2930, 1645, 1550, 1080 $cm^{-1}$ |
| $^1$H-NMR ($D_2O$): | δ 1,75 (dt, 2H, 6Hz); 3,27 (t, 4H, 6Hz); 3,4 - 4,1 (m, 20H); 4,15 (t, 2H, 3Hz); 4,39 (d, 2H, 3Hz); 4,54 (d, 2H, 7Hz); 4,70 ($H_2O$, i.St.) |
| $^{13}$C-NMR ($D_2O$): | δ 30,79; 38,99; 63,73; 64,65; 71,30; 73,12; 73,74; 74,14; 74,50; 75,06; 75,18; 77,99; 83,71; 106,10; 176,84 i. St.: $CH_3OH$ δ 51,56 |

Beispiel 4

N,N'-1,6-Hexandiylbis[4-O-β-D-galactopyranosyl-D-gluconamid]

Man suspendiert 17,0 g Lactobionsäure-1,5-lacton in 100 ml aminfreiem Dimethylformamid, versetzt mit 2,9 g 1,6-Diaminohexan und rührt 6 Stunden bei 80 °C. Nach Abkühlen wird filtriert, und das Filtrat in 1 l Diethylether eingerührt. Der teilweise ölige Niederschlag wird in 50 ml Wasser gelöst und mit 80 ml Ionenaustauscher (Merck 4765, $H^+$-Form) behandelt. Man filtriert und erhält nach Lyophilisierung 19,5 g farbloses Pulver, das sich ab 175 °C unter Braunfärbung zersetzt.

IR: v = 2930, 2860, 1645, 1548, 1080 $cm^{-1}$

$^1$H-NMR ($D_2O$): δ 1,0 - 1,8 (m, 8H); 3,25 (t, 4H, 5,5Hz); 3,3 - 4,1 (m, 20H); 4,15 (t, 2H, 3Hz); 4,38 (d, 2H, 3Hz); 4,55 (d, 2H, 7Hz); 4,70 ($H_2O$)
i. St.: 3-Trimethylsilyl-propansulfonsäure-Na-Salz

$^{13}$C-NMR ($D_2O$): δ 28,19; 30,89; 41,63; 63,68; 64,64; 71,25; 73,06; 73,72; 74,12; 74,96; 75,18; 77,97; 83,61; 106,08; 176,42 i. St.: $CH_3OH$ δ 51,54

Beispiel 5

N,N'-1,12-Dodecandiylbis[4-O-β-D-galactopyranosyl-D-gluconamid]

Man suspendiert 40,8 g Lactobionsäure-1,5-lacton in 150 ml aminfreiem Dimethylformamid, gibt 12,0 g 1,12-Diaminododecan zu und rührt 6 Stunden bei 60 °C. Unter Rühren tropft man das Gemisch in 1,5 l Isopropanol. Der Niederschlag wird mit Isopropanol gewaschen und in 250 ml Wasser gelöst. Man behandelt die Lösung erst mit 20 ml eines sauren Ionenaustauschers (Lewatit S 100), dann mit einem basischen Ionenaustauscher (Merck 4767). Nach Lyophilisierung erhält man 35,0 g eines farblosen Pulvers. Schmelzpunkt: 79 - 81 °C

IR: v = 2920, 2850, 1645, 1550, 1080 $cm^{-1}$

$^1$H-NMR ($D_2O$): δ 0,8 - 1,9 (m, 20H); 3,24 (t, 4H, 5,5Hz); 3,4 - 4,1 (m, 20H); 4,17 (t, 2H, 3Hz); 4,38 (d, 2H, 3Hz); 4,55 (d, 2H, 7Hz); 4,68 ($H_2O$, i. St.)

$^{13}$C-NMR ($D_2O$): δ 29,02; 31,36; 31,70; 41,86; 63,64; 64,69; 71,20; 73,08; 73,72; 74,17; 75,03; 75,20; 77,97; 83,72; 106,12; 176,24; i. St. $CH_3OH$ δ 51,56

Beispiel 6

N,N'-1,9-Nonandiylbis [4-O-β-D-galactopyranosyl-D-gluconamid]

Herstellung und Reinigung analog Beispiel 5. Man erhält aus 15,0 g Lactobionsäure-1,5-lacton und 3,47 g 1,9-Diaminononan 15,0 g der Titelverbindung.

IR: v = 2930, 2860, 1660, 1545, 1080 $cm^{-1}$

$^1$H-NMR ($D_2O$): δ 0,9 - 1,8 (m, 14H); 3,20 (t, 4H, 5,5Hz); 3,3 - 4,1 (m, 20H); 4,15 (t, 2H, 3Hz); 4,38 (d, 2H, 3Hz); 4,55 (d, 7Hz); 4,68 ($H_2O$, i.St.)

Beispiel 7

N,N'-1,12-Dodecandiylbis (4-O-β-D-glucopyranosyl-D-gluconamid)

Man setzt 2,04 g Cellobionsäure-1,5-lacton (H. W. Diehl et al., Carbohydr. Res. 38, 364 (1974)) analog Beispiel 5 mit 0,60 g 1,12-Diaminododecan um und erhält 0,60 g der Titelverbindung.

IR: v = 2925; 2850, 1645, 1545, 1075, 1040 $cm^{-1}$

$^1$H-NMR ($D_2O$): δ 0,7 - 1,9 (m, 20H); 3,0 - 4,6 (m, 30H); 4,68 ($H_2O$ i. St.)

Beispiel 8

N,N'-1,12-Dodecandiylbis (4-O-α-D-glucopyranosyl-D-gluconamid)

Man setzt 20,0 g Calcium-maltobionat (W.N. Emmerling, B. Pfannemüller, Starch 33, 202, (1981)) analog Beispiel 3 mit 1,12-Diaminododecan um und erhält 17,8 g Produkt.

IR: v = 2925, 2850, 1650, 1545, 1145, 1075, 1030 $cm^{-1}$

$^1H$-NMR ($D_2O$): δ 0,7 - 1,9 (m, 20H); 3,20 (t, 4H, 5,5Hz); 3,3 - 4,4 (m, 24H); 5,15 (d, 2H, 3Hz); 4,68 ($H_2O$, i.St.)

Beispiel 9

N,N'-1,12-Dodecandiylbis (6-O-α-D-galactopyranosyl)-D-gluconamid)

Man setzt 3,96 g Kalium-melibionat (Sigma-Chemie) analog Beispiel 3 mit 1,00 g 1,12-Diaminododecan um und erhält 3,3 g der Titelverbindung.

Schmelzpunkt: 114 - 123 °C
IR (KBr): ν = 2925, 1855, 1645, 1550, 1150, 1080, 1030, 980 $cm^{-1}$
$^1H$-NMR ($D_2O$): δ 0,8 - 1,8 (m, 20H); 3,20 (m, 4H); 3,4 - 4,2 (m, 22H), 4,29 (d, 2H, 3Hz); 4,95 (s, 2H); 4,68 ($H_2O$, i. St.)

Beispiel 10

N,N'-1,3-Propandiylbis (6-O-α-D-galactopyranosyl-D-gluconamid)

Herstellung analog Beispiel 9. Aus 3,96 g Kalium-melibionat und 0,37 g 1,3-Diaminopropan erhält man 3,0 g Produkt.

Schmelzpunkt: 90 - 96 °C
IR (KBr): ν = 2925, 1645, 1550, 1152, 1080, 1030, 975 $cm^{-1}$
$^1H$-NMR ($D_2O$): δ 1,76 (dt, 2H, 6,5Hz); 3,30 (t, 4H, 6,5 Hz); 3,4 - 4,2 (m, 22H); 4,33 (d, 2H, 3Hz); 4,96 (s, 2H); 4,70 ($H_2O$, i. St.)
$^{13}C$-NMR ($D_2O$): δ 30,73; 38,96; 63,75; 70,94; 74,13; 71,90; 72,12; 73,06; 73,52; 74,52; 75,97; 100,99; 176,91

Beispiel 11

N,N'-α,α'-m-Xyloldiylbis[4-O-β-D-galactopyranosyl-D-gluconamid]

Verwendet man bei einem Verfahren gemäß Beispiel 5 17,0 g Lactobionsäure-1,5-lacton und 3,3 ml 3-(Aminomethyl)-benzylamin, so erhält man auf gleiche Weise 12,2 g der Titelverbindung als farbloses Pulver.

IR: ν = 2920, 1665, 1545, 1080 $cm^{-1}$
$^1H$-NMR ($D_2O$): δ 3,3 - 4,6 (m, 30H); 4,68 ($H_2O$); 7,24 (m, 4H)

Beispiel 12

N,N'-4,4'-Dicyclohexylmethandiylbis[4-O-β-D-galactopyranosyl-D-gluconamid]

Herstellung und Reinigung analog Beispiel 5 aus 17,0 g Lactobionsäure-1,5-lactonund 5,3 g 4,4'-Diamino-dicyclohexylmethan. Ausbeute: 21,3 g.

IR: ν = 2930, 2850, 1645, 1545, 1080 $cm^{-1}$
$^1H$-NMR ($D_2O$): δ 0,6 - 2,2 (m, 20H); 3,2 - 4,6 (m, 28H), 4,68 ($H_2O$)

Beispiel 13

N,N'-1,6-(3,4-Dithiahexandiylbis)4-O-β-D-galactopyranosyl-D-gluconamid

Man versetzt 17,0 g Lactobionsäure-1,5-lacton und 5,63 g Cystamin Dihydrochlorid in 50 ml aminfreiem DMF bei Raumtemperatur mit 6,9 ml Triethylamin und rührt anschließend 6 Stunden bei 60 °C. Man fällt mit 500 ml Ethanol und behandelt den Niederschlag wie in Beispiel 5 weiter. Man erhält 13,2 g weißes Pulver.

IR: ν = 2925, 1650, 1545, 1080 $cm^{-1}$
$^1H$-NMR ($D_2O$): δ 2,90 (t, 4H, 6Hz); 3,2 - 4,1 (m, 24H); 4,16 (t, 2H, 3Hz); 4,38 (d, 2H, 3Hz); 4,55 (d, 2H, 7 Hz); 4,68 ($H_2O$, i. St.)

Beispiel 14

N,N'-1,7-(4-Azaheptandiylbis)4-O-β-D-galactopyranosyl-D-gluconamid

Man suspendiert 17,0 g Lactobionsäure-1,5-lacton in 100 ml aminfreiem Dimethylformamid, versetzt bei Raumtemperatur mit 2,28 ml Bis-(3-aminopropyl)-amin und rührt 10 Stunden. Dann rührt man 4 Stunden bei 40 °C und filtriert. Das Filtrat rührt man in 900 ml Aceton ein und erhält nach Waschen mit Aceton und Trocknen 23,0 g weiße Kristalle. Diese werden in 80 ml Wasser gelöst und mit 900 ml Aceton gefällt. Der teils ölige Niederschlag wird in 150 ml Wasser gelöst, filtriert und lyophilisiert. Ausbeute: 16,5 g.

IR: v = 2920, 1650, 1545, 1080 $cm^{-1}$

$^1$H-NMR ($D_2O$): δ 1,82 (dt, 4H, 6Hz); 2,91 (t, 4H, 6Hz); 3,30 (t, 4H, 6Hz); 3,45 - 4,6 (m, 26H); 4,68 ($H_2O$)

Beispiel 15

N,N'-1,12-(4,9-Dioxadodecandiylbis)4-O-β-D-galactopyranosyl-D-gluconamid

Herstellung und Reinigung analog Beispiel 5. Man erhält aus 17,0 g Lactobionsäure-1,5-lacton und 5,1 g 1,12-Diamino-4,9-dioxa-dodecan 18,9 g der Titelverbindung.

$^1$H-NMR: δ 1,4 - 2,0 (m, 8H); 3,1 - 4,1 (m, 32H); 4,6 (t, 2H, 3Hz); 4,38 (d, 2H, 3Hz); 4,55 (d, 2H, 7Hz)

Beispiel 16

N,N'-Dimethyl-N,N'-1,2-ethandiylbis (4-O-β-D-galactopyranosyl-D-gluconamid)

Herstellung und Reinigung analog Beispiel 5. Man erhält aus 3,40 g Lactobionsäure-1,5-lacton und 0,44 g N,N'-Dimethylethylendiamin 3,0 g der Titelverbindung.

Schmelzpunkt: 125 - 133 °C

IR (KBr): v = 2930, 1640, 1400, 1075 $cm^{-1}$

$^1$H-NMR ($D_2O$): δ 2,99, 316 (2S, 6H); 3,3 - 4,3 (m, 28H); 4,49 (d, 2H, 7Hz); 4,68 ($H_2O$, i. St.)

Beispiel 17

N,N'-1,5-(1-Ethoxycarbonyl)-pentandiylbis (4-O-β-D-galactopyranosyl-D-gluconamid)

Man suspendiert 2,47 g Lysinethylester-Dihydrochlorid in 40 ml aminfreiem Dimethylformamid, versetzt mit 3,0 ml Triethylamin und rührt 15 Minuten. Dann gibt man 6,8 g Lactobionsäure-1,5-lacton zu, erwärmt auf 60 °C und rührt 1 Tag. Man filtriert und rührt das Filtrat in 400 ml Isopropanol ein. Der Niederschlag wird gesammelt, in 60 ml Dimethylformamid gelöst und erneut mit 300 ml Isopropanol gefällt. Man wiederholt die Fällung, wäscht mit Isopropanol und Diethylether und erhält so 4,05 g eines weißen Pulvers.

Schmelzpunkt: 106 °C

IR: v = 2930, 1735, 1655, 1550, 1075 $cm^{-1}$

$^1$H-NMR ($D_2O$): δ 1,25 (t, 3H, 7Hz); 1,2 - 2,2 (m, 6H); 3,25 (t, 2H, 5,5Hz); 3,4 - 4,6 (m, 29H); 4,68 ($H_2O$, i. St.)

Beispiel 18

Decanatrium N,N'-1,3-propandiylbis (2,3,4,5,6-penta-O-sulfo-D-gluconamid)

4,30 g N,N'-1,3-Propandiylbis-D-gluconamid werden in 50 ml trockenem Dimethylformamid suspendiert, auf 40 °C erwärmt und unter Rühren mit 23,9 g Pyridin-Schwefeltrioxid-Komplex versetzt. Nach wenigen Minuten fällt das Produkt in Form des Pyridiniumsalzes als Öl aus. Nach 1 Stunde läßt man abkühlen und dekantiert die überstehende Lösung ab. Das Öl wird in 50 ml Wasser gelöst und mit 6 N Natronlauge auf pH = 10 gebracht. Die Lösung wird mit Wasser auf 90 ml aufgefüllt und in 350 ml einer 1 %igen Natriumacetatlösung eingerührt. Der Niederschlag wird mit Methanol gewaschen und getrocknet. Man erhält 18,6 g eines farblosen Pulvers. Dieses löst man in 186 ml Wasser. In die Lösung rührt man 227 ml Methanol ein und läßt 15 Stunden stehen. Man dekantiert vom abgeschiedenen Öl und verreibt dieses mit Methanol. Man wiederholt die Fällung, bis die Titelverbindung rein vorliegt. Zersetzung ab 190 °C unter

Braunfärbung.

IR (KBr): ν = 2960, 1670, 1555, 1250, 1073, 1045, 1019, 770 $cm^{-1}$

$^1$H-NMR ($D_2O$): δ 1,87 (dt, 2H, 7Hz); 3,36 (dt, 4H, 7Hz); 3,9 - 4,6 (m, 4H); 4,8 - 5,4 (m, 8H); 4,68 ($H_2O$, i. St.)

$[\alpha]_{20}^{D}$ = +26,2 (c = 5 in $H_2O$)

| Elementaranalyse: | | |
|---|---|---|
| ber.: | N 22,10 % | S 1,93 % |
| gef.: | N 22,29 % | S 1,83 % |

$^{13}$C-NMR ($D_2O$): δ 29,97; 39,69; 69,06; 77,68; 78,10; 78,39; 79,65; 171,33; i. St.: $CH_3OH$ δ 51,56

Beispiel 19

Decanatrium N,N'-1,12-dodecandiylbis (2,3,4,5,6-penta-D-sulfo-D-gluconamid)

Man setzt 5,60 g N,N'-1,12-Dodecandiylbis-D-gluconamid analog Beispiel 18 mit 25,5 g Pyridin-Schwefeltrioxid-Komplex um und erhält 20,5 g rohes Produkt. Das reine Produkt gewinnt man durch Gelchromatographie einer wässrigen Lösung an einer Sephadex G 25-Säule. Nach Gefriertrocknung erhält man ein farbloses Pulver, das sich zwischen 173 °C und 189 °C unter Braunfärbung zersetzt.

IR (KBr): ν = 2930, 2855, 1665, 1555, 1250, 1072, 1042, 1010 $cm^{-1}$

$^1$H-NMR ($D_2O$): δ 1,0 - 1,9 (m, 20H); 3,32 (m, 4H); 4,2 - 4,6 (m, 4H); 4,9 - 5,3 (m, 8H); 4,68 ($H_2O$, i.St.)

$^{13}$C-NMR ($D_2O$): 28,72; 30,52; 31,02; 42,30; 69,22; 77,67; 78,34; 78,65; 79,91; 171,09; i. St. $CH_3OH$ δ 51,56

Beispiel 20

Hexadecanatrium N,N'-1,3-propandiylbis[2,3,5,6-tetra-O-sulfo-4-O-(2,3,4,6-tetra-O-sulfo-β-D-galactopyranosyl)-D-gluconamid]

Man löst 79,1 g Calciumlactobionat in 240 ml Wasser und behandelt die Lösung mit 240 ml Lewatit S 100 ($H^+$-Form). Man wäscht den Ionenaustauscher mit 3 x 200 ml Wasser und engt die vereinigten Lösungen so weit wie möglich ein. Der glasartige Rückstand wird in 700 ml aminfreiem Dimethylformamid gelöst und mit 600 ml n-Hexan am Wasserabscheider zum Sieden erhitzt. Nach beendeter Wasserabscheidung dampft man das n-Hexan ab und versetzt die Lösung bei Raumtemperatur mit 7,7 g 1,3-Diaminopropan in 50 ml Dimethylformamid. Nach 5stündigem Rühren bei 60 °C läßt man auf ca. 30 °C abkühlen, verdünnt mit 450 ml Dimethylformamid und gibt rasch portionsweise unter Rühren 400 g Pyridin-Schwefeltrioxid-Komplex zu. Man rührt 1 Stunde zwischen 40 und 45 °C und läßt abkühlen. Man dekantiert vom abgeschiedenen Öl, löst dieses in 500 ml Wasser und stellt die Lösung mit 30 %iger Natronlauge auf pH = 10 ein. Man ergänzt mit Wasser auf ein Volumen von 1,5 l und rührt die Lösung in 4,5 l einer 1 %igen methanolischen Natriumacetatlösung ein. Der Niederschlag wird mit 1 l Methanol verrührt, abgesaugt und getrocknet. Man erhält 250 g eines gelblichen Pulvers. Dieses löst man in 2 l Wasser, versetzt mit 250 ml 30 %igem Hydrogenperoxid und rührt 1 Stunde bei 45 °C. Nach dem Abkühlen neutralisiert man und ergänzt mit Wasser auf 2,5 l. Die Lösung rührt man in 3,06 l Methanol ein und läßt 15 Stunden stehen. Man dekantiert von abgeschiedenem Öl und verreibt dieses mit Methanol. Nach dem Trocknen erhält man 188,5 g farbloses Pulver. Man wiederholt den Fällungsvorgang viermal und erhält schließlich ca. 50 g der reinen Titelverbindung als farbloses Pulver, das sich ab 172 °C unter Zersetzung braun färbt und nicht unter 250 °C schmilzt.

IR (KBr): ν = 2965, 1665, 1552, 1250, 1055, 1020, 927, 820 $cm^{-1}$

$^1$H-NMR ($D_2O$): δ 1,82 (t, 2H, 6,5Hz); 3,35 (t, 4H, 6,5Hz); 3,9 - 4,4 (m, 8H); 4,4 - 4,8 (m, + $H_2O$-Signal bei 4,68 als i. St.); 4,8 - 5,4 (m, 10H)

$^{13}$C-NMR ($D_2O$): δ 30,31; 39,77; 68,36; 68,92; 74,22, 77,49; 77,79; 78,39; 78,76; 80,15; 103,55; 171,76; i. St.: $CH_3OH$ δ 51,56

$[\alpha]_{20}^{D}$ = + 13,3° (c = 5 in $H_2O$)

| Elementaranalyse: | | |
|---|---|---|
| ber.: | N: 1,17 % | S: 21,49 % |
| gef.: | N: 1,16 % | S: 21,61 % |

Beispiel 21

Pentadecanatrium-pentadeca-O-sulfo-N,N'-1,3-propandiylbis (4-O-β-D-galactopyranosyl-D-gluconamid)

Man löst 3,77 g N,N'-1,3-Propandiylbis (4-O-β-D-galactopyranosyl-D-gluconamid) in 60 ml trockenem Dimethylformamid und versetzt bei 40 °C unter Rühren portionsweise mit 13,5 g Pyridin-Schwefeltrioxid-Komplex.Nach 1 Stunde arbeitet man wie in Beispiel 18 auf und erhält 10,3 g gelbliches, sulfathaltiges Rohprodukt. Man löst in 90 ml Wasser, versetzt mit 10 ml 30 %igem Hydrogenperoxid und rührt 1 Stunde bei 45 °C. Nach Abkühlen rührt man 230 ml Methanol ein und läßt 15 Stunden stehen. Man dekantiert vom abgeschiedenen Öl ab, verreibt dieses mit Methanol und erhält 6,72 g sulfatfreies Produkt (mit einem Schwefelgehalt von 20,6 %). Man löst in 67 ml Wasser, rührt 82 ml Methanol ein und läßt 15 Stunden stehen. Man dekantiert vom abgeschiedenen Öl und rührt in den Überstand weitere 74 ml Methanol ein. Nach 15 Stunden isoliert man das Öl und wiederholt damit mehrmals die fraktionierte Fällung wie oben, bis die Titelverbindung rein vorliegt. Man erhält 0,53 g farbloses Pulver, das sich ab 180 °C unter Braunfärbung zersetzt.

IR (KBr): ν = 2960, 1660, 1550, 1250, 1055, 1020, 930, 820 $cm^{-1}$

$^1$H-NMR ($D_2O$): δ 1,87 (t, 2H, 6Hz); 3,42 (t, 4H, 6Hz); 3,9 - 4,5 (m, 8H); 4,5 - 4,85 (m + $H_2O$-Signal bei 4,68 als i. St.); 4,85 - 5,3 (m, 10H)

$^{13}$C-NMR ($D_2O$): δ 30,53; 39,79; 68,46; 69,11; 72,28; 74,36; 74,56; 77,43; 77,88; 78,14; 78,49; 79,03; 79,61; 79,84; 80,43; 103,45; 171,82; 172,61; i. St.: $CH_3OH$ δ 51,56

| Elementaranalyse: | | |
|---|---|---|
| ber.: | N 1,23 % | S 21,04 % |
| gef.: | N 1,21 % | S 20,91 % |

Beispiel 22

Hexadecamorpholinium N,N'-1,3-propandiylbis[2,3,5,6-tetra-O-sulfo-4-O-(2,3,4,6-tetra-O-sulfo-β-D-galactopyranosyl)-D-gluconamid]

Man behandelt eine Lösung von 1,76 g des Natriumsalzes aus Beispiel 20 15 Minuten mit 16 ml Lewatit S-100 ($H^+$-Form), filtriert den Ionenaustauscher ab und versetzt das Filtrat mit 1,03 g Morpholin. Nach Lyophilisierung erhält man 2,40 g gelbliches Pulver.

Zersetzung ab 120 °C und Schwarzfärbung bei 210 °C

IR (KBr): ν = 2950, 2780, 1665, 1563, 1450, 1426, 1250, 1097, 1015, 925, 893, 868, 810 $cm^{-1}$

$^1$H-NMR ($D_2O$): δ 1,82 (dt, 2H; 6,5Hz); 3,15 (m, 64H); 3,35 (m, 4H); 3,90 (m, 64H); 4,0 - 4,4 (m, 8H); 4,4 - 4,8 (m, + $H_2O$-Signal bei 4,70 als i. St.); 4,8 - 5,4 (m, 10H)

Beispiel 23

Hexadecanatrium N,N'-1,6-hexandiylbis[2,3,5,6-tetra-O-sulfo-4-O-(2,3,4,6-tetra-O-sulfo-β-D-galactopyranosyl)-D-gluconamid]

Man setzt 16,3 g N,N'-1,6-Hexandiylbis (4-O-β-D-galactopyranosyl-D-gluconamid) mit 75,0 g Pyridin-Schwefeltrioxid-Komplex analog Beispiel 18 um. Nach der ersten Fällung erhält man 56,9 g eines gelblichen Pulvers, das man wie in Beispiel 20 reinigt. Man gelangt schließlich zu ca. 15 g der reinen Titelverbindung in Form eines farblosen Pulvers, das ab 120 °C sintert.

Zersetzung ab 170 °C unter Braunfärbung.

IR (KBr): ν = 2930, 2860, 1655, 1550, 1250, 1055, 1020, 928, 810 $cm^{-1}$

$^{1}H$-NMR ($D_2O$): δ 1,1 - 1,9 (m, 8H); 3,37 (m, 4H); 3,9 - 4,5 (m, 8H); 4,5 - 4,85 (m + $H_2O$-Signal bei 4,68 als i. St.); 4,85 - 5,3 (m, 10H)
$^{13}C$-NMR ($D_2O$): δ 28,42; 30,74; 42,17; 68,56; 69,01; 74,39; 77,20; 77,80; 78,37; 78,94; 80,47; 103,21; 171,27; i. St. $CH_3OH$ δ 51,56
$[\alpha]_{20}^{D}$ = + 9,9 (c = 5 in $H_2O$)

Beispiel 24

Hexadecanatrium N,N'-1,9-nonandiylbis[2,3,5,6-tetra-O-sulfo-4-O-(2,3,4,6-tetra-O-sulfo-β-D-galactopyranosyl)-D-gluconamid]

Herstellung und Reinigung analog Beispiel 23. Aus 15,0 g N,N'-1,9-Nonandiylbis(4-O-β-D-galactopyranosyl-D-gluconamid) und 63,0 g Pyridin-Schwefeltrioxid-Komplex erhält man 45,0 g Rohprodukt. Nach Reinigung: 10,5 g farbloses Pulver.

Zersetzung zwischen 192 - 210 °C unter Braunfärbung
IR (KBr): ν = 2935, 2860, 1665, 1555, 1250, 1057, 1020, 925, 815 $cm^{-1}$
$^{1}H$-NMR ($D_2O$): δ 0,9 - 1,9 (m, 14H); 3,29 (t, 4H, 6,5Hz); 3,8 - 4,45 (m, 8H); 4,45 - 4,8 (m + $H_2O$-Signal bei 4,68 als i. St.); 4,8 - 5,4 (m, 10H)
$^{13}C$-NMR ($D_2O$): δ 28,77; 30,83; 31,09; 31,32; 42,19; 68,69; 68,99; 74,46; 77,12; 77,79; 78,33; 78,93; 80,51; 103,11; 121,21 i. St.: $CH_3OH$, δ 51,56

Beispiel 25

HexadecanatriumN,N'-1,12-dodecandiylbis[2,3,5,6-tetra-O-sulfo-4-O-(2,3,4,6-tetra-O-sulfo-β-D-galactopyranosyl)-D-gluconamid]

Herstellung und Reinigung analog Beispiel 23. Aus 4,23 g N,N'-1,12-Dodecandiylbis(4-O-β-D-galactopyranosyl-D-gluconamid) und 19,1 g Pyridin-Schwefeltrioxid-Komplex erhält man 13,30 g Rohprodukt. Nach Reinigung: 3,5 g reine Titelverbindung als farbloses Pulver.

Zersetzung zwischen 188 - 198 °C unter Braunfärbung
IR (KBr): ν = 2940, 2880, 1665, 1555, 1250, 1055, 1020, 930, 820 $cm^{-1}$
$^{1}H$-NMR ($D_2O$): δ 0,9 - 1,9 (m, 20H); 3,35 (t, 4H, 6,5Hz); 3,9 - 4,5 (m, 8H); 4,5 - 4,8 (m, +$H_2O$-Signal bei 4,70 als i. St.); 4,8 - 5,4 (m, 10H)
$^{13}C$-NMR ($D_2O$): δ 28,74; 30,80; 31,09; 31,44; 42,18; 68,76; 68,96; 74,50; 77,08; 77,80; 78,29; 78,94; 80,51; 103,07; 171,19 i. St.: $CH_3OH$ δ 51,56

Beispiel 26

Hexadecanatrium N,N'-1,12-dodecandiylbis[2,3,5,6-tetra-O-sulfo-4-O-(2,3,4,6-tetra-O-sulfo-β-D-glucopyranosyl)-D-gluconamid]

Aus 0,34 g N,N'-1,12-Dodecandiylbis (4-O-β-D-glucopyranosyl-D-gluconamid)und 1,12 g Pyridin-Schwefeltrioxid-Komplex erhält man analog Beispiel 23 0,68 g rohes bzw. 0,10 g reines Produkt.

Zersetzung ab 148 °C bis 159 °C unter Braunfärbung.
IR (KBr): ν = 2930, 2855, 1670, 1560, 1250, 1070, 995, 935, 800 $cm^{-1}$
$^{1}H$-NMR ($D_2O$): δ 0,8 - 1,8 (m, 20H); 3,30 (m, 4H); 3,7 - 4,8 (m + $H_2O$-Signal bei 4,68 als i. St.); 4,8 - 5,3 (m, 10H)
$^{13}C$-NMR ($D_2O$): δ 28,92; 30,95; 31,32; 31,64; 42,34; 69,20; 70,12; 75,57; 77,44; 77,67; 77,85; 79,33; 79,41; 79,97; 81,08; 102,53; 171,27; i. St.: $CH_3OH$ δ 51,56

Beispiel 27

Hexadecanatrium N,N'-1,12-dodecandiylbis[2,3,5,6-tetra-O-sulfo-4-O-(2,3,4,6-tetra-O-sulfo-α-D-glucopyranosyl)-D-gluconamid]

Aus 12,8 g N,N'-1,12-Dodecandiylbis (4-O-α-D-glucopyranosyl-D-gluconamid) und 64,6 g Pyridin-Schwefeltrioxid-Komplex erhält man analog Beispiel 23 47,5 g rohes bzw. 3,0 g reines Produkt.

Zersetzung von 175 °C bis 189 °C unter Braunfärbung.

IR (KBr): ν = 2930, 2860, 1660, 1560, 1250, 1000, 943, 805 $cm^{-1}$

$^{1}H$-NMR ($D_2O$): δ 1,0 - 1,9 (m, 20H); 3,27 (m, 4H); 4,0 - 4,82 (m + Signal für $H_2O$ bei 4,68 als i. St.); 4,82 - 5,25 (m, 10H); 5,52 (d, 2H, 3Hz)

$^{13}C$-NMR ($D_2O$): 28,84; 30,69; 31,15; 31,47; 42,41; 68,51; 69,29; 71,95; 76,14; 76,82; 77,91; 78,30; 78,44; 79,98; 98,93; 171,27

Beispiel 28

Hexadecanatrium N,N'-1,12-dodecandiylbis[2,3,4,5-tetra-O-sulfo-6-O-(2,3,4,6-tetra-O-sulfo-α-D-galactopyranosyl)-D-gluconamid]

Analog Beispiel 23 erhält man aus 3,30 g N,N'-1,12-Dodecandiylbis (6-O-α-D-galactopyranosyl-D-gluconamid) und 14,9 g Pyridin-Schwefeltrioxid-Komplex 9,7 g rohes bzw. 3,4 g reines Prodkt, das ab 57 °C sintert.

Zersetzung ab 182 °C unter Braunfärbung.

IR (KBr): ν = 2930, 2855, 1650, 1555, 1250, 1050, 1027, 830 $cm^{-1}$

$^{1}H$-NMR ($D_2O$): δ 1,0 - 1,9 (m, 20H); 3,25 (m, 4H); 3,9 - 4,4 (m, 8H); 4,4 - 4,8 (m + $H_2O$-Signal bei 4,68 als i. St.); 4,8 - 5,25 (m, 10H); 5,38 (d, 2H, 3Hz)

$^{13}C$-NMR ($D_2O$): δ 28,72; 30,58; 31,03; 31,34; 42,30; 69,14; 69,77; 70,66; 74,51; 74,92; 77,91; 78,21; 78,49; 78,93; 80,75; 99,12; 171,26; i. St.: $CH_3OH$ δ 51,56

Beispiel 29

Hexadecanatrium N,N'-1,3-propandiylbis[2,3,4,5-tetra-O-sulfo-6-O-(2,3,4,6-tetra-O-sulfo-α-D-galactopyranosyl)-D-gluconamid]

Aus 0,34 g N,N'-1,3-Propandiylbis (6-O-α-D-galactopyranosyl-D-gluconamid) und 2,0 g Pyridin-Schwefeltrioxid-Komplex erhält man analog Beispiel 23 0,96 g rohes bzw. 0,50 g reines Produkt.

Zersetzung ab 168 °C unter Braunfärbung

IR (KBr): ν = 1640, 1550, 1250, 1050, 1025, 830 $cm^{-1}$

$^{1}H$-NMR ($D_2O$): δ 1,85 (t, 2H, 6,5Hz); 3,35 (t, 4H, 6,5 Hz); 3,9 - 4,4 (m, 8H); 4,4 - 4,8 (m + $H_2O$-Signal bei 4,68 als i. St.); 4,8 - 5,25 (m, 10H); 5,36 (d, 2H, 3Hz)

$^{13}C$-NMR ($D_2O$): δ 30,13; 39,84; 69,17; 69,86; 70,74; 74,53; 74,97; 78,00; 78,17; 78,37; 79,00; 80,81; 99,18; 171,70; i. St.: $CH_3OH$ δ 51,57

Beispiel 30

Hexadecanatrium N,N'-α,α'-m-xyloldiylbis[2,3,5,6-tetra-O-sulfo-4-O-(2,3,4,6-tetra-O-sulfo-β-D-galactopyranosyl)-D-gluconamid]

Aus 12,0 g N,N'-α,α'-m-Xyloldiylbis(4-O-β-D-galactopyranosyl-D-gluconamid) und 58,8 g Pyridin-Schwefeltrioxid-Komplex erhält man analog Beispiel 23 28,0 g rohes bzw. 5,3 g reines Produkt.

Zersetzung ab 157 °C unter Braunfärbung.

IR (KBr): ν = 2960, 1660, 1550, 1250, 1055, 1020, 930, 815 $cm^{-1}$

$^{1}H$-NMR ($D_2O$): δ 3,9 - 4,85 (m + $H_2O$-Signal bei 4,68 als i. St.), 4,85 - 5,4 (m, 10H); 7,38 (s, 4H)

$^{13}C$-NMR ($D_2O$): δ 45,51; 68,63; 69,15; 74,42; 77,24; 77,67; 77,91; 78,49; 79,08; 80,70; 103,29; 128,15; 128,86; 131,64; 140,80; 171,88; i. St. $CH_3OH$, δ 51,56

Beispiel 31

Hexadecanatrium N,N'-4,4-dicyclohexylmethandiylbis[2,3,5,6-tetra-O-sulfo-4-O-(2,3,4,6-tetra-O-sulfo-β-D-galactopyranosyl)-D-gluconamid]

Analog Beispiel 23 erhält man aus 25,7 g N,N'-4,4'-Dicyclohexylmethandiylbis (4-O-β-D-galactopyranosyl-D-gluconamid) und 114,7 g Pyridin-Schwefeltrioxid-Komplex 70,7 g rohes bzw. 15,2 g reines Produkt, das ab 120 °C sintert.

Zersetzung ab 180 °C unter Braunfärbung.

IR (KBr): ν = 2930, 2860, 1660, 1550, 1250, 1055, 1020, 928, 815 $cm^{-1}$

$^{1}H$-NMR ($D_2O$): δ 0,6 - 2,4 (m, 20H); 3,65 (m, 2H); 3,9 - 4,5 (m, 8H); 4,5 - 4,85 (m + $H_2O$-Signal bei 4,68 als i. St.); 4,85 - 4,4 (m, 10H)

$^{13}C$-NMR ($D_2O$): δ 30,18; 30,36; 30,75; 34,09; 44,40; 46,20; 49,45; 52,33; 68,27; 68,75; 74,35; 77,80; 78,41; 78,68; 79,49; 104,09; 170,61; i. St. $CH_3OH$ δ 51,56

$[\alpha]_{20}^{D}$ = + 10,0 (c = 5 in $H_2O$)

Beispiel 32

Hexadecanatrium N,N'-1,6-(3,4-dithiahexandiylbis)[2,3,5,6-tetra-O-sulfo-4-O-(2,3,4,6-tetra-O-sulfo-β-D-galactopyranosyl)-D-gluconamid]

Aus 11,2 g N,N'-1,6-(3,4-Dithiahexandiylbis)4-O-β-D-galactopyranosyl-D-gluconamid) und 53,4 g Pyridin-Schwefeltrioxid-Komplex erhält man analog Beispiel 23 38,0 g rohes und 8,5 g reines Produkt.

Zersetzung ab 163 °C unter Braunfärbung.

IR (KBr): ν = 2965, 1665, 1550, 1250, 1055, 1015, 930, 810 $cm^{-1}$

$^{1}H$-NMR ($D_2O$): δ 2,96 (t, 4H, 6,5Hz); 3,69 (m, 4H); 4,0 - 4,47 (m, 8H); 4,45 - 4,8 (m + $H_2O$-Signal bei 4,68 als i. St.); 4,8 - 5,3 (m, 10H)

$^{13}C$-NMR ($D_2O$): δ 38,72; 41,06; 68,68; 69,05; 74,48; 77,40; 77,87; 78,46; 80,46; 103,48; 171,86; i. St. $CH_3OH$ δ 51,56

Beispiel 33

Hexadecanatrium N,N'-1,7-(4-azaheptandiylbis)[2,3,5,6-tetra-O-sulfo-4-O-(2,3,4,6-tetra-O-sulfo-β-D-galactopyranosyl)-D-gluconamid]

Aus 11,0 g N,N'-1,7-(4-Azaheptandiylbis)4-O-β-D-galactopyranosyl-D-gluconamid und 50,0 g Pyridin-Schwefeltrioxid-Komplex erhält man analog Beispiel 23 15,4 g rohes und 2,2 g reines Produkt.

Zersetzung ab 165 °C unter Braunfärbung.

IR (KBr): ν = 2960, 2925, 2855, 1650, 1550, 1250, 1055, 1020, 927, 820 $cm^{-1}$

$^{1}H$-NMR ($D_2O$): δ 2,98 (m, 2H); 3,17 (t, 4H, 7Hz); 3,44 (t, 4H, 6Hz); 3,9 - 4,4 (m, 8H); 4,4 - 4,85 (m + $H_2O$-Signal als i. St. bei 4,68); 4,85 - 5,3 (m, 10H)

$^{13}C$-NMR ($D_2O$): δ 28,11; 39,08; 48,12; 68,65; 69,24; 74,45; 76,94; 77,93; 78,46; 79,09; 80,71; 103,13; 172,06

Beispiel 34

Hexadecanatrium N,N'-1,12-(4,9-dioxadodecandiylbis)[2,3,5,6-tetra-O-sulfo-4-O-(2,3,4,6-tetra-O-sulfo-β-D-galactopyranosyl)-D-gluconamid]

Aus 18,2 g N,N'-1,12-(4,9-dioxadodecandiylbis)4-O-β-D-galactopyranosyl-D-gluconamid und 59,0 g Pyridin-Schwefeltrioxid-Komplex erhält man gemäß Beispiel 23 37,1 g rohes und 9,3 g reines Produkt, das ab 120 °C sintert.

Zersetzung ab 170 °C unter Braunfärbung.

IR (KBr): ν = 2960, 2880, 1665, 1555, 1250, 1055, 1022, 928, 815 $cm^{-1}$

$^{1}H$-NMR ($D_2O$): δ 1,64 (m, 4H); 1,88 (t, 4H, 6,5Hz); 3,0 - 3,9 (m, 12H); 3,9 - 4,45 (m, 8H); 4,45 - 4,8 (m + $H_2O$-Signal bei 4,68 als i. St.); 4,8 - 5,3 (m, 10H)

$^{13}C$-NMR ($D_2O$): δ 27,82; 30,78; 39,02; 68,64; 69,01; 70,54; 72,96; 74,44; 77,02; 77,79; 78,33; 78,94; 80,52; 103,10; 171,45; i. St.: $CH_3OH$ δ 51,56

$[\alpha]_{20}^{D}$ = + 9,0 (c = 5 in $H_2O$)

Beispiel 35

Hexadecanatrium N,N'-dimethyl-N,N'-1,2-ethandiylbis[2,3,5,6-tetra-O-sulfo-4-O-(2,3,4,6-tetra-O-sulfo-β-D-galactopyranosyl)-D-gluconamid]

Aus 2,50 g N,N'-Dimethyl-N,N'-1,2-ethandiylbis (4-O-β-D-galactopyranosyl-D-gluconamid) und 12,4 g Pyridin-Schwefeltrioxid-Komplex erhält man analog Beispiel 23 8,2 g rohes und 1,2 g reines Produkt.

Zersetzung von 188 - 200 °C unter Braunfärbung.

IR (KBr): v = 2970, 1650, 1250, 1015, 930, 815 $cm^{-1}$

$^1$H-NMR($D_2O$): δ 3,0 - 4,0 (m mit s bei 3,35; 10H); 4,0 - 4,7 (m, 14H); 4,70 ($H_2O$, i. St.); 4,9 - 5,4 (m, 10H); 5,54 (d, 2H, 4Hz)

$^{13}$C-NMR ($D_2O$): δ 38,96; 48,28; 68,36; 69,25; 74,17; 75,11; 77,26; 77,73; 78,00; 78,45; 78,76; 79,80; 103,35; 171,25; i. St.: $CH_3OH$, δ 51,56

Beispiel 36

Hexadecanatrium N,N'-1,5-(1-ethoxycarbonyl)-pentandiylbis[2,3,5,6-tetra-O-sulfo-4-O-(2,3,4,6-tetra-O-sulfo-β-D-galactopyranosyl)-D-gluconamid]

Aus 3,6 g N,N'-1,5-(1-Ethoxycarbonyl)-pentandiylbis(4-O-β-D-galactopyranosyl-D-gluconamid) und 15,8 g Pyridin-Schwefeltrioxid-Komplex erhält man gemäß Beispiel 23 8,7 g rohes und 1,2 g reines Produkt, das ab 60 °C sintert.

Zersetzung ab 161 °C unter Braunfärbung.

IR (KBr): v = 1730, 1650, 1550, 1250, 1055, 1020, 930, 810 $cm^{-1}$

$^1$H-NMR($D_2O$): δ 1,0 - 2,2 (m, 9H, mit t bei 1,31, 7 Hz); 3,30 (m, 2H); 3,9 - 4,8 (m mit $H_2O$-Signal bei 4,68 als i. St.); 4,8 - 5,3 (m, 10H)

$^{13}$C-NMR ($D_2O$): δ 15,94; 29,68; 30,54; 33,17; 41,87; 55,93; 65,19; 68,23; 68,53; 69,04; 74,36; 77,33; 79,81; 78,45; 78,80; 79,61; 80,47; 103,36; 103,99; 171,39; 171,65; 176,12

Beispiel 37

N,N'-1,3-Propandiylbis-D-gulonamid

Man löst 3,56 g D-Gulono-γ-lacton und 0,84 ml 1,3-Diaminopropan in 40 ml Dimethylformamid und rührt 6 Stunden bei 60 °C. Nun rührt man das Gemisch in 200 ml Isopropanol ein und wäscht den Niederschlag mit Isopropanol und Diethylether. Man löst den Feststoff in 20 ml Dimethylformamid und fällt nochmals mit 203 ml Isopropanol. Der Niederschlag wird in Wasser gelöst und gefriergetrocknet. Man erhält 2,2 g eines farblosen Pulvers.

Schmelzpunkt: v = 2930, 2890, 1645, 1545, 1440, 1080 $cm^{-1}$

$^1$H-NMR ($D_2O$): δ 1,74 (dt, 2H, 6,5Hz); 3,27 (t, 4H, 6,5Hz); 3,45 - 4,05 (m, 10H); 4,23 (d, 2H, 6Hz); 4,68 ($H_2O$, i. St.)

$^{13}$C-NMR ($D_2O$): δ 30,65; 39,02; 65,13; 72,64; 74,69; 75,00; 75,12; 176,78; i. St. $CH_3OH$ δ 51,56

Beispiel 38

N,N'-1,2-Propandiylbis-D-galactonamid

Analog Beispiel 37 erhält man aus 7,12 g D-Gelactono-γ-lacton und 1,48 g 1,2-Diaminopropan 4,1 g der Titelverbindung als farbloses Pulver.

Schmelzpunkt: 183 - 193 °C unter Zersetzung und Braunfärbung

IR (KBr): v = 2940, 1656, 1552, 1109, 1055, 1044, 1028, 865 $cm^{-1}$

$^1$H-NMR ($D_2O$): δ 1,18 (d, 3H, 6Hz); 3,1 - 4,6 (m, 15H); 4,68 ($H_2O$, i. St.)

$^{13}$C-NMR ($D_2O$): δ 19.64; 19.77; 46.16; 47.87; 48.10; 65.90; 71.93; 72.64; 73.49; 177.75; 178.42; 178.54

Beispiel 39

N,N'-1,4-Butandiylbis-L-mannonamid

Analog Beispiel 18 erhält man aus 3,56 g L-Mannono-γ-lacton und 0,90 g Putrescin 2,4 g der Titelverbindung als farbloses Pulver.

Zersetzung von 181 - 188 °C unten Braunfärbung

| | |
|---|---|
| IR (KBr): | ν = 2955, 2925, 2855, 1643, 1555, 1231, 1098, 1043, 1031, 880, 740, 640 $cm^{-1}$ |
| $^{1}$H-NMR ($D_2O$): | δ 1.58 (m, 4H); 3.30 (m, 4H); 3.75 (m, 8H) 4.02 (d, 2H, 7Hz); 4.26 (d, 2H, 7Hz); 4.68 ($H_2O$ i. St.) |
| $^{13}$C-NMR ($D_2O$): | δ 28.42; 41.38; 65.67; 72.58; 72.76; 73.43; 75.19; 177.09; |

Beispiel 40

N,N'-Dilactobionoylhydrazin

Analog Beispiel 37 erhält man aus 6,8 g Lactobionsäure-1,5-lacton und 0,5 ml Hydrazinhydrat 6,1 g Rohprodukt Säulenchromatographie über Fractogel TSK HW 4OS liefert das reine Produkt als farbloses Pulver nach Gefriertrocknung.

Beispiel 41

Decanatrium N,N'-1,3-propandiylbis (2,3,4,5,6-penta-O-sulfo-D-gulonamid)

Analog Beispiel 18 erhält man aus 2,2 g N,N'-1,3-Propandiylbis-D-gulonamid und 12,3 g Pyridin-Schwefeltrioxid-Komplex 9,8 g rohes bzw. 6,4 g reines Produkt als farbloses Pulver.

Zersetzung ab 185 °C unter Braunfärbung.

| | |
|---|---|
| IR (KBr): | ν = 2960, 1675, 1555, 1250, 1070, 1010, 925, 805 $cm^{-1}$ |
| $^{1}$H-NMR ($D_2O$): | δ 1,85 (m, 2H); 3,34 (m, 4H); 4,52 (d, 4H, 3,5Hz) 5,07 (m, 6H); 5,34 (d, 2H, 3,5 Hz); 4,68 ($H_2O$, i. St.) |
| $^{13}$C-NMR ($D_2O$): | δ 30.05; 39.62; 68.78; 76.28; 76.41; 77.78; 80.14; 171.15; i. St. $CH_3OH$ δ 51.55 |

Beispiel 42

Decanatrium N,N'-1,2-propandiylbis (2,3,4,5,6-penta-O-sulfo-D-galactonamid)

Analog Beispiel 18 erhält man aus 3,3 g N,N'-1,2-Propandiylbis-D-galactonamid und 19,5 g Pyridin-Schwefeltrioxid-Komplex 13,0 g rohes bzw. 9,8 g reines Produkt als farbloses Pulver.

Zersetzung ab 191 °C unter Braunfärbung.

| | |
|---|---|
| IR (KBr): | ν = 2970, 1665, 1550, 1250, 1065, 1040, 1007, 900 $cm^{-1}$ |
| $^{1}$H-NMR ($D_2O$): | δ 1,26 (d, 3H, 6,5Hz); 2,9 - 4,3 (m, 3H); 4,3 - 4,6 (m, 4H); 4,68 ($H_2O$, i. St.); 4,8 - 5,3 (m, 8H) |
| $^{13}$C-NMR ($D_2O$): | δ 19.20; 45.94; 46.15; 47.61; 69.07; 78.42; 78.86; 79.90; 170.84; 171.03; 191.93 i. St. $CH_3OH$ δ 51.57 |

Beispiel 43

Decanatrium N,N'-1,4-butandiylbis(2,3,4,5,6-penta-O-sulfo-L-mannonamid)

Analog Beispiel 18 erhalt man aus 2,5 g N,N'-1,4-Butandiylbis-L-mannonamid und 14,1 g Pyridin-Schwefeltrioxid-Komplex 10,5 g rohes bzw. 7,2 g reines Produkt als farbloses Pulver.

Zersetzung ab 180 °C unter Braunfärbung

| | |
|---|---|
| IR (KBr): | ν = 2960, 2930, 2850, 1670, 1555, 1250, 1075, 1010, 925 $cm^{-1}$ |
| $^{1}$H-NMR ($D_2O$): | δ 1.65 (m, 4H); 3.31 (m, 4H); 4.43 (m, 4H); 4.8-5.08 (m, 4H); 5.15 (m, 4H); 4.68 ($H_2O$, i. St.) |
| $^{13}$C-NMR ($D_2O$): | δ 27.98; 41.62; 69.15; 78.81; 79.36; 79.75; 170.93; i. St. $CH_3OH$ δ 51.55 |

Beispiel 44

Hexadecanatrium N,N'-bis[2,3,5,6-tetra-O-sulfo-4-O-(2,3,4,6-tetra-O-sulfo-β-D-galactopyranosyl)-gluconoyl]-hydrazin

Analog Beispiel 18 erhält man aus 6,0 g N,N'-Dilactobionoylhydrazin und 33,7 g Pyridin-Schwefeltrioxid-Komplex 17,5 g rohes bzw. 7,3 g reines Produkt.

Beispiel 45

Man löst bei Zimmertemperatur 5,000 kg Trockensubstanz von Hexadecanatrium N,N'-1,3-propandiylbis[2,3,5,6-tetra-O-sulfo-4-O-(2,3,4,6-tetra-O-sulfo-β-D-galactopyranosyl)-D-gluconamid] unter Rühren in 40 l aqua ad iniectabilia. Nach Einstellen des pH-Wertes der Lösung auf 7,5 mit verdünnter Natronlauge ergänzt man mit aqua ad iniectabilia auf 50,00 l und filtriert durch ein Membranfilter der Porenweite 0,2 µm. Die Lösung wird unter aseptischen Bedingungen in Ampullen zu 1 ml abfiltriert und diese abgeschmolzen.

**Patentansprüche**

**Patentansprüche für folgende Vertragsstaaten : AT, BE, CH, LI, DE, FR, GB, IT, LU, NL, SE**

**1.** Polyschwefelsäureester von Bis-aldonsäureamiden und deren Derivaten der allgemeinen Formel I

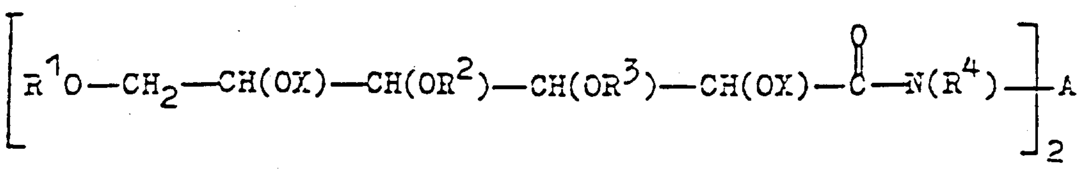

(I)

worin entweder
sämtliche Reste $R^1$, $R^2$ und $R^3$ unabhängig voneinander für X stehen, oder
zwei der Reste $R^1$, $R^2$ und $R^3$ für X, und der dritte für einen Rest der Formeln II - VII stehen,

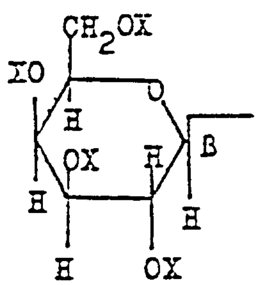

(II)

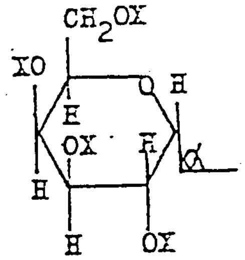

(III)

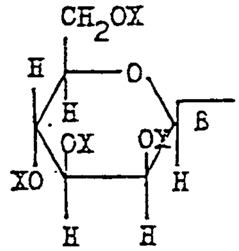

(IV)

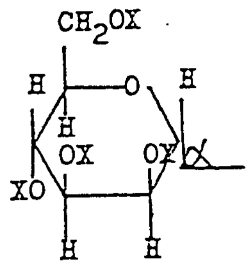

(V)

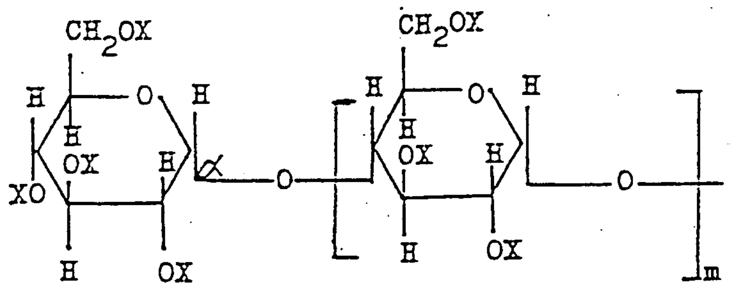

(VI)

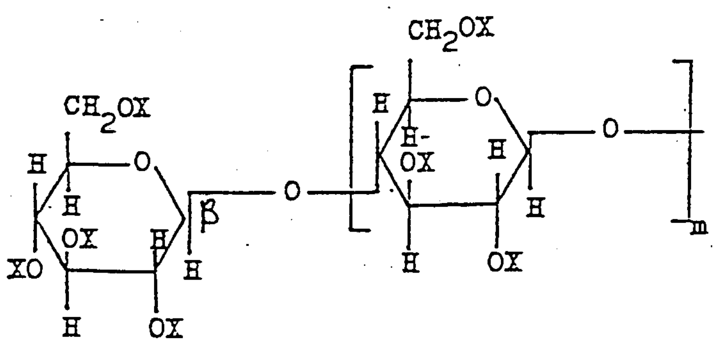

(VII)

X in den Formeln I bis VII gleichzeitig oder unabhängig voneinander ein Wasserstoffatom oder die Gruppe $-SO_3H$ bedeutet, wobei mindestens ein X für die Gruppe $-SO_3H$ steht.

m für 0,1,2,3,4,5 oder 6 steht,

A in Formel I für einen gegebenenfalls durch einen oder mehrere Reste $-CO_2R^5$ substituierten geradkettigen oder verzweigten, gesättigten Alkylenrest mit 2 bis 22

Kohlenstoffatomen steht, und dieser Alkylenrest gegebenenfalls durch bis zu 5 -O-, -S-, -S-S-, $-S(O)_n-$,

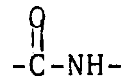

oder/und $-NR^6$-Gruppen oder Cycloalkylen- oder Arylenreste unterbrochen ist, oder A für eine einfache Bindung oder den Rest

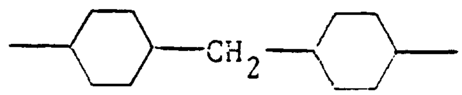

steht,

n 1 oder 2 ist,

$R^4$, $R^5$ und $R^6$ gleichzeitig oder unabhängig voneinander ein Wasserstoffatom oder einen $C_1$-$C_6$-Alkylrest bedeuten,

sowie deren Salze mit anorganischen oder organischen Basen.

**2.** Verbindungen nach Anspruch 1, dadurch gekennzeichnet, daß jedes X in den Formeln I bis VII für die Gruppe $-SO_3H$ steht.

**3.** Verbindungen nach Anspruch 1, dadurch gekennzeichnet, daß in den Formeln I bis VII mindestens die Hälfte der vorhandenen Gruppen X eine $-SO_3H$-Gruppe darstellt.

**4.** Verbindungen nach Anspruch 1, dadurch gekennzeichnet, daß $R^2$ und $R^3$ für X stehen.

**5.** Verbindungen nach Anspruch 4, dadurch gekennzeichnet, daß $R^1$ den Rest III oder den Rest VI mit m = 0 bedeutet.

**6.** Verbindungen nach Anspruch 1, dadurch gekennzeichnet, daß $R^1$ und $R^3$ für X stehen.

**7.** Verbindungen nach Anspruch 6, dadurch gekennzeichnet, daß $R^2$ den Rest II oder den Rest VI mit m = 0 oder den Rest VII mit m = 0 bedeutet.

**8.** Verbindungen nach einem der Ansprüche 4 bis 7, dadurch gekennzeichnet, daß A in Formel I einen Polymethylenrest $-(CH_2)_p-$ mit p = 2 bis 22 bedeutet.

**9.** Verbindungen nach einem der Ansprüche 4 bis 7, dadurch gekennzeichnet, daß A in der Formel I einen Polymethylenrest $-(CH_2)_p-$ mit p = 2 bis 12 bedeutet.

**10.** Verbindungen nach einem der Ansprüche 4 bis 7, dadurch gekennzeichnet, daß A in Formel I einen geradkettigen Alkylenrest mit 2 bis 22 Kohlenstoffatomen darstellt, dessen Kette durch die Gruppen -O-, -S-, -S-S-, $-S(O)_n-$,

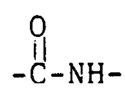

und/oder $-NR^6$- unterbrochen sein kann, wobei n und $R^6$ die in Anspruch 1 angegebenen Bedeutungen besitzen.

**11.** Verbindungen nach einem der Ansprüche 8 bis 10, dadurch gekennzeichnet, daß A in Formel I durch einen, zwei oder mehrere Reste $-CO_2R^5$ substituiert ist, wobei $R^5$ die in Anspruch 1 angegebene Bedeutung hat.

**12.** Verfahren zur Herstellung von Polyschwefelsäureestern von Bis-aldonsäureamiden und deren Derivaten der Formel I gemäß Anspruch 1, dadurch gekennzeichnet, daß man Bis-aldonsäureamide der allgemeinen Formel IX,

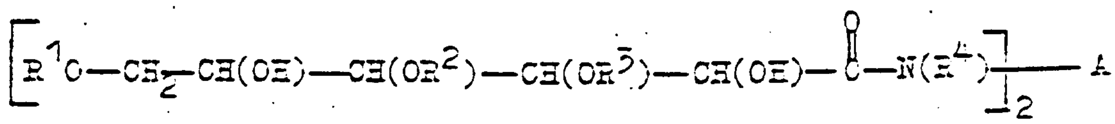

worin $R^1$, $R^2$, $R^3$, $R^4$ und A, die in Anspruch 1 angegebenen Bedeutungen haben, wobei jedoch X in den Formeln II bis VII für ein Wasserstoffatom steht, in einem aprotischen Lösungsmittel mit einem Sulfatierungsmittel umsetzt und die so erhaltenen Produkte gegebenenfalls mit einer anorganischen oder organischen Base in die entsprechenden Salze überführt.

**13.** Verfahren nach Anspruch 12, dadurch gekennzeichnet, daß man jeweils, ohne Isolierung von Zwischenstufen, Aldonsäuren der allgemeinen Formel VIII,

$R^1O—CH_2—CH(OH)—CH(OR^2)—CH(OR^3)—CH(OH)—CO_2H$ (VIII)

worin $R^1$, $R^2$ und $R^3$ die in Anspruch 1 angegebenen Bedeutungen haben, wobei jedoch X in den Formeln II bis VII für ein Wasserstoffatom steht, in die Bis-aldonsäureamide der allgemeinen Formel IX überführt und zu deren Polyschwefelsäureestern umsetzt.

**14.** Arzneimittel, enthaltend eine oder mehrere Verbindungen der allgemeinen Formel I, gemäß einem der Ansprüche 1 bis 11, zusammen mit üblichen Träger- und Hilfsstoffen.

**Patentansprüche für folgenden Vertragsstaat : ES**

**1.** Verfahren zur Herstellung von Polyschwefelsäureestern von Bis-aldonsäureamiden und deren Derivaten der allgemeinen Formel I

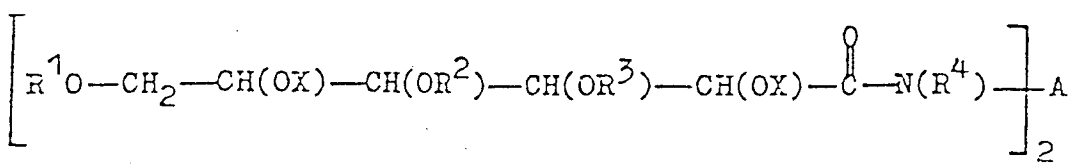 (I)

worin entweder
sämtliche Reste $R^1$, $R^2$ und $R^3$ unabhängig voneinander für X stehen, oder
zwei der Reste $R^1$, $R^2$ und $R^3$ für X, und der dritte für einen Rest der Formeln II - VII stehen,

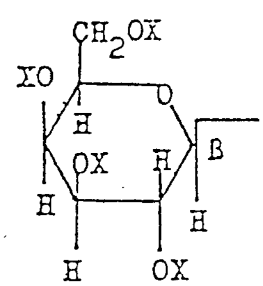 (II)

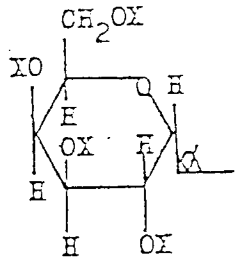

(III)

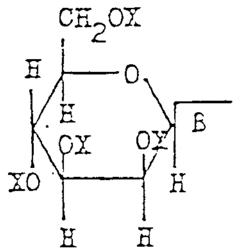

(IV)

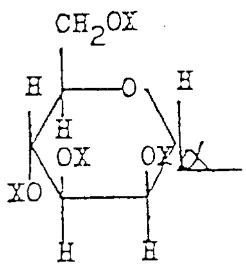

(V)

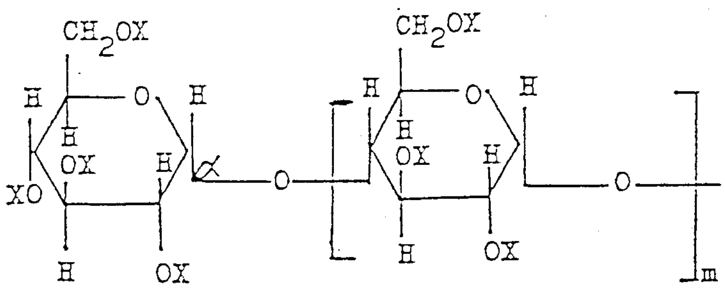

(VI)

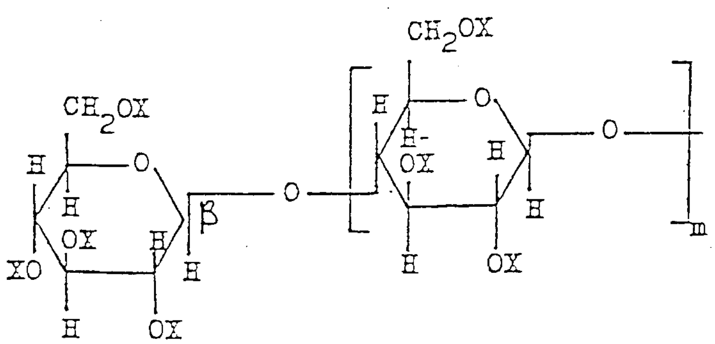

(VII)

X in den Formeln I bis VII gleichzeitig oder unabhängig voneinander ein Wasserstoffatom oder die Gruppe $-SO_3H$ bedeutet, wobei mindestens ein X für die Gruppe $-SO_3H$ steht,

m für 0,1,2,3,4,5 oder 6 steht,

A in Formel I für einen gegebenenfalls durch einen oder mehrere Reste $-CO_2R^5$ substituierten geradkettigen oder verzweigten, gesättigten Alkylenrest mit 2 bis 22

Kohlenstoffatomen steht, und dieser Alkylenrest gegebenenfalls durch bis zu 5 -O-, -S-, -S-S-, $-S(O)_n-$,

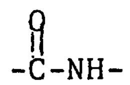

oder/und $-NR^6$-Gruppen oder Cycloalkylen- oder Arylenreste unterbrochen ist, oder A für eine einfache Bindung oder den Rest

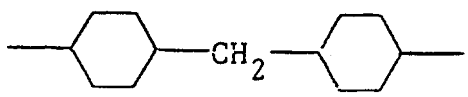

steht,

n — 1 oder 2 ist,

$R^4$, $R^5$ und $R^6$ — gleichzeitig oder unabhängig voneinander ein Wasserstoffatom oder einen $C_1$-$C_6$-Alkylrest bedeuten,

sowie von deren Salzen mit anorganischen oder organischen Basen, dadurch gekennzeichnet, daß man Bis-aldonsäureamide der allgemeinen Formel IX

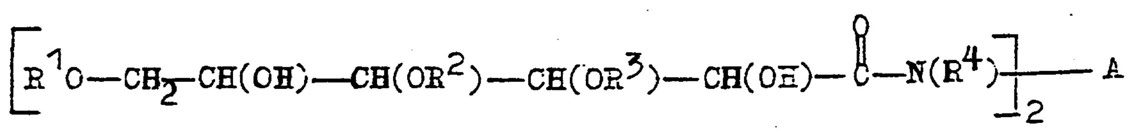

(IX)

worin $R^1$, $R^2$, $R^3$, $R^4$ und A, die vorstehend angegebenen Bedeutungen haben, wobei jedoch X in den Formeln II bis VII für ein Wasserstoffatom steht, in einem aprotischen. Lösungsmittel mit einem Sulfatierungsmittel umsetzt und die so erhaltenen Produkte gegebenenfalls mit einer anorganischen oder organischen Base in die entsprechenden Salze überführt.

**2.** Verfahren nach Anspruch 1 , dadurch gekennzeichnet, daß man jeweils, ohne Isolierung von Zwischenstufen, Aldonsäuren der allgemeinen Formel VIII,

$$R^1O—CH_2—CH(OH)—CH(OR^2)—CH(OR^3)—CH(OH)—CH_2H \qquad (VIII)$$

worin $R^1$, $R^2$ und $R^3$ die in Anspruch 1 angegebenen Bedeutungen haben, wobei jedoch X in den Formeln II bis VII für ein Wasserstoffatom steht, in die Bis-aldonsäureamide der allgemeinen Formel IX überführt und zu deren Polyschwefelsäureestern umsetzt.

**3.** Verfahren nach Anspruch 1 oder 2, dadurch gekennzeichnet, daß jedes X in den Formeln I bis VII für die Gruppe $-SO_3H$ steht.

**4.** Verfahren nach Anspruch 1 oder 2, dadurch gekennzeichnet, daß in den Formeln I bis VII mindestens die Hälfte der vorhandenen Gruppen X eine $-SO_3H$-Gruppe darstellt.

**5.** Verfahren nach Anspruch 1 oder 2, dadurch gekennzeichnet, daß $R^2$ und $R^3$ für X stehen.

**6.** Verfahren nach Anspruch 5 , dadurch gekennzeichnet, daß $R^1$ den Rest III oder den Rest VI mit m = 0 bedeutet.

**7.** Verfahren nach Anspruch 1 oder 2, dadurch gekennzeichnet, daß $R^1$ und $R^3$ für X stehen.

8. Verfahren nach Anspruch 7 , dadurch gekennzeichnet, daß $R^2$ den Rest II oder den Rest VI mit m = 0 oder den Rest VII mit m = 0 bedeutet.

9. Verfahren nach einem der Ansprüche 5 bis 8, dadurch gekennzeichnet, daß A in Formel I einen Polymethylenrest $-(CH_2)_p-$ mit p = 2 bis 22 bedeutet.

10. Verfahren nach einem der Ansprüche 5 bis 8 , dadurch gekennzeichnet, daß A in der Formel I einen Polymethylenrest $-(CH_2)_p-$ mit p = 2 bis 12 bedeutet.

11. Verfahren nach einem der Ansprüche 5 bis 8, dadurch gekennzeichnet, daß A in Formel I einen geradkettigen Alkylenrest mit 2 bis 22 Kohlenstoffatomen darstellt, dessen Kette durch die Gruppen -O-, -S-, -S-S-, $-S(O)_n-$,

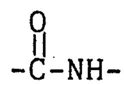

und/oder $-NR^6-$ unterbrochen sein kann, wobei n und $R^6$ die in Anspruch 1 angegebenen Bedeutungen besitzen.

12. Verfahren nach einem der Ansprüche 9 bis 11 , dadurch gekennzeichnet, daß A in Formel I durch einen, zwei oder mehrere Reste $-CO_2R^5$ substituiert ist, wobei $R^5$ die in Anspruch 1 angegebene Bedeutung hat.

**Patentansprüche für folgenden Vertragsstaat : GR**

1. Verfahren zur Herstellung von Polyschwefelsäureestern von Bis-aldonsäureamiden und deren Derivaten der allgemeinen Formel I

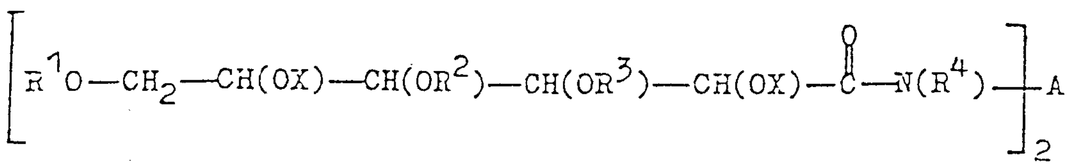
(I)

worin entweder
sämtliche Reste $R^1$, $R^2$ und $R^3$ unabhängig voneinander für X stehen, oder
zwei der Reste $R^1$, $R^2$ und $R^3$ für X, und der dritte für einen Rest der Formeln II - VII stehen,

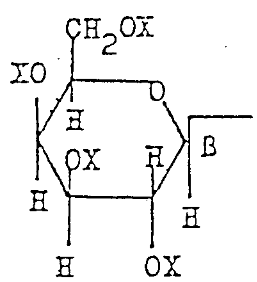
(II)

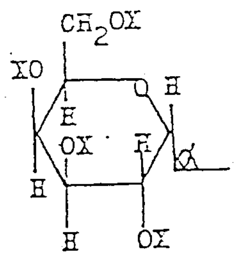

(III)

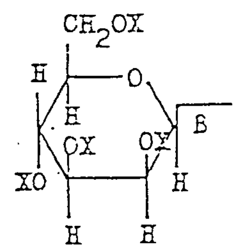

(IV)

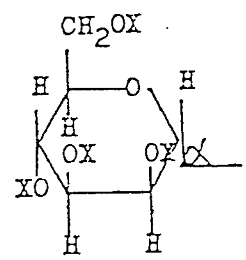

(V)

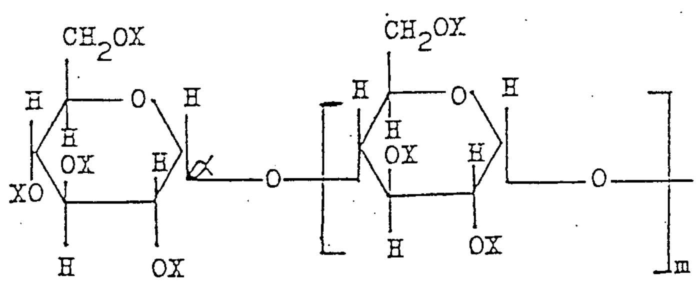

(VI)

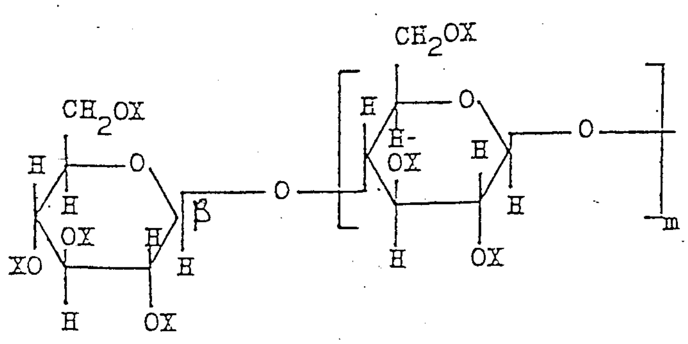

(VII)

X in den Formeln I bis VII gleichzeitig oder unabhängig voneinander ein Wasserstoffatom oder die Gruppe $-SO_3H$ bedeutet, wobei mindestens ein X für die Gruppe $-SO_3H$ steht,

m für 0,1,2,3,4,5 oder 6 steht,

A in Formel I für einen gegebenenfalls durch einen oder mehrere Reste $-CO_2R^5$ substituierten geradkettigen oder verzweigten, gesättigten Alkylenrest mit 2 bis 22 Kohlenstoffatomen steht, und dieser Alkylenrest gegebenenfalls durch bis zu 5

-O-, -S-, -S-S-, $-S(O)_n-$,

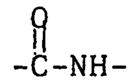

oder/und $-NR^6$-Gruppen oder Cycloalkylen- oder Arylenreste unterbrochen ist, oder A für eine einfache Bindung oder den Rest

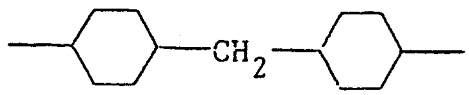

steht,

n 1 oder 2 ist,

$R^4$, $R^5$ und $R^6$ gleichzeitig oder unabhängig voneinander ein Wasserstoffatom oder einen $C_1$-$C_6$-Alkylrest bedeuten,

sowie von deren Salzen mit anorganischen oder organischen Basen, dadurch gekennzeichnet, daß man Bis-aldonsäureamide der allgemeinen Formel IX

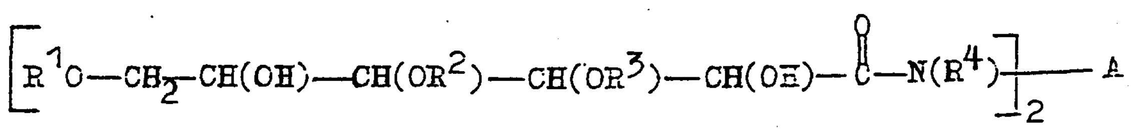

(IX)

worin $R^1$, $R^2$, $R^3$, $R^4$ und A, die vorstehend angegebenen Bedeutungen haben, wobei jedoch X in den Formeln II bis VII für ein Wasserstoffatom steht, in einem aprotischen Lösungsmittel mit einem Sulfatierungsmittel umsetzt und die so erhaltenen Produkte gegebenenfalls mit einer anorganischen oder organischen Base in die entsprechenden Salze überführt.

2. Verfahren nach Anspruch 1 , dadurch gekennzeichnet, daß man jeweils, ohne Isolierung von Zwischenstufen, Aldonsäuren der allgemeinen Formel VIII,

$$R^1O-CH_2-CH(OH)-CH(OR^2)-CH(OR^3)-CH(OH)-CO_2H \quad (VIII)$$

worin $R^1$, $R^2$ und $R^3$ die in Anspruch 1 angegebenen Bedeutungen haben, wobei jedoch X in den Formeln II bis VII für ein Wasserstoffatom steht, in die Bis-aldonsäureamide der allgemeinen Formel IX überführt und zu deren Polyschwefelsäureestern umsetzt.

3. Verfahren nach Anspruch 1 oder 2, dadurch gekennzeichnet, daß jedes X in den Formeln I bis VII für die Gruppe $-SO_3H$ steht.

4. Verfahren nach Anspruch 1 oder 2, dadurch gekennzeichnet, daß in den Formeln I bis VII mindestens die Hälfte der vorhandenen Gruppen X eine $-SO_3H$-Gruppe darstellt.

5. Verfahren nach Anspruch 1 oder 2, dadurch gekennzeichnet, daß $R^2$ und $R^3$ für X stehen.

6. Verfahren nach Anspruch 5 , dadurch gekennzeichnet, daß $R^1$ den Rest III oder den Rest VI mit m = 0 bedeutet.

7. Verfahren nach Anspruch 1 oder 2, dadurch gekennzeichnet, daß $R^1$ und $R^3$ für X stehen.

8. Verfahren nach Anspruch 7, dadurch gekennzeichnet, daß $R^2$ den Rest II oder den Rest VI mit m = 0 oder den Rest VII mit m = 0 bedeutet.

**9.** Verfahren nach einem der Ansprüche 5 bis 8, dadurch gekennzeichnet, daß A in Formel I einen Polymethylenrest $-(CH_2)_p-$ mit p = 2 bis 22 bedeutet.

**10.** Verfahren nach einem der Ansprüche 5 bis 8, dadurch gekennzeichnet, daß A in der Formel I einen Polymethylenrest $-(CH_2)_p-$ mit p = 2 bis 12 bedeutet.

**11.** Verfahren nach einem der Ansprüche 5 bis 8, dadurch gekennzeichnet, daß A in Formel I einen geradkettigen Alkylenrest mit 2 bis 22 Kohlenstoffatomen darstellt, dessen Kette durch die Gruppen -O-, -S-, -S-S-, $-S(O)_n-$,

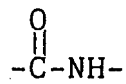

und/oder $-NR^6-$ unterbrochen sein kann, wobei n und $R^6$ die in Anspruch 1 angegebenen Bedeutungen besitzen.

**12.** Verfahren nach einem der Ansprüche 9 bis 11 , dadurch gekennzeichnet, daß A in Formel I durch einen, zwei oder mehrere Reste $-CO_2R^5$ substituiert ist, wobei $R^5$ die in Anspruch 1 angegebene Bedeutung hat.

**13.** Verwendung von Polyschwefelsäureestern von Bis-aldonsäureamiden und deren Derivaten der Formel I, wie in Anspruch 1 definiert, sowie von deren Salzen mit anorganischen oder organischen Basen zur Herstellung von Arzneimitteln.

**Claims**
**Claims for the following Contracting States : AT, BE, CH, LI, DE, FR, GB, IT, LU, NL, SE**

**1.** Polysulphuric acid esters of bis-aldonamides and their derivatives of the general formula I

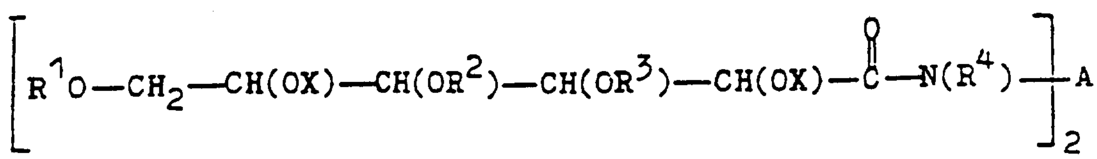

(I)

in which either
all radicals $R^1$, $R^2$ and $R^3$ independently of one another stand for X, or
two of the radicals $R^1$, $R^2$ and $R^3$ stand for X, and the third stands for a radical of the formulae II-VII

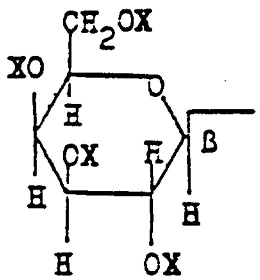

(II)

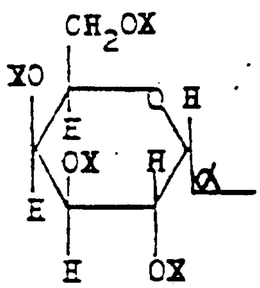

(III)

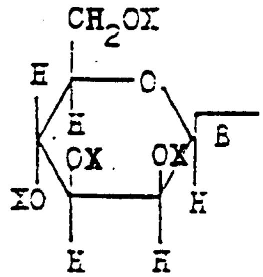

(IV)

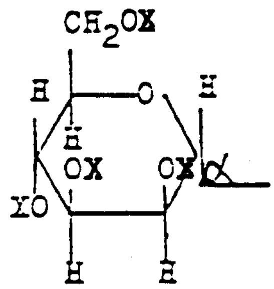

(V)

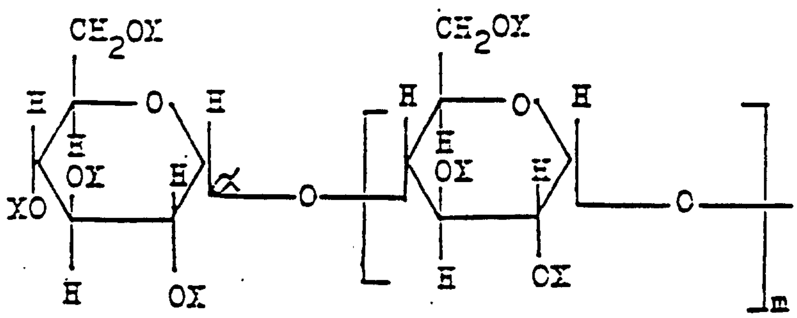

(VI)

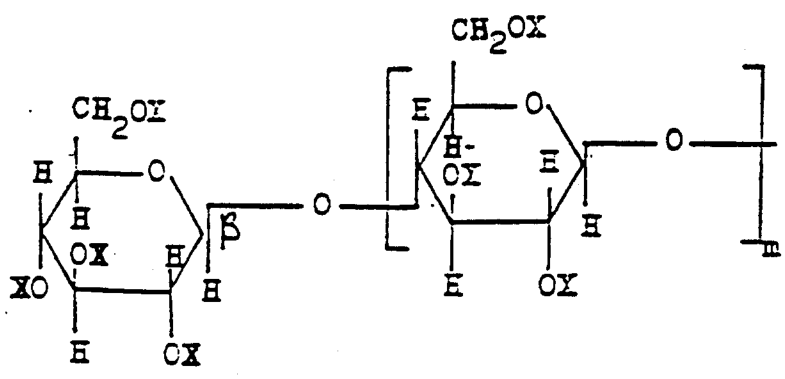

(VII)

X in the formulae I to VII simultaneously or independently of one another denotes a hydrogen atom or the group $-SO_3H$, where at leat one X stands for the group $-SO_3H$,
m stands for 0, 1, 2, 3, 4, 5 or 6,
A in formula I stands for a straight-chain or branched, saturated alkylene radical having 2 to 22 carbon atoms which is optionally substituted by one or more radicals $-CO_2R^5$, and this alkylene radical is optionally interrupted by up to 5 -O-, -S-, -S-S-, $-S(O)_n-$,

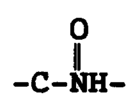

or/and $-NR^6$ groups or cycloalkylene or arylene radicals, or A stands for a single bond or the radical

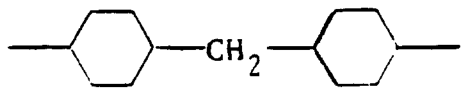

n is 1 or 2,

$R^4$, $R^5$ and $R^6$ simultaneously or independently of one another denote a hydrogen atom or a $C_1$-$C_6$-alkyl radical,

and their salts with inorganic or organic bases.

2. Compounds according to Claim 1, characterised in that each X in the formulae I to VII stands for the group $-SO_3H$.

3. Compounds according to Claim 1, characterised in that in the formulae I to VII at least half the X groups present represent an $-SO_3H$ group.

4. Compounds according to Claim 1, characterised in that $R\omega 2$ and $R^3$ stand for X.

5. Compounds according to Claim 4, characterised in that $R^1$ denotes the radical III or the radical VI having m = 0.

6. Compounds according to Claim 1, characterised in that $R^1$ and $R^3$ stand for X.

7. Compounds according to Claim 6, characterised in that $R^2$ denotes the radical II or the radical VI having m = 0 or the radical VII having m = 0.

8. Compounds according to one of Claims 4 to 7, characterised in that A in formula I denotes a polymethylene radical $-(CH_2)_p-$ having p = 2 to 22.

9. Compounds according to one of Claims 4 to 7, characterised in that A in formula I denotes a polymethylene radical $-(CH_2)_p-$ having p = 2 to 12.

10. Compounds according to one of Claims 4 to 7, characterised in that A in formula I represents a straight-chain alkylene radical having 2 to 22 carbon atoms, whose chain can be interrupted by the groups -O-, -S-, -S-S-, $-S(O)_n-$,

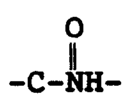

and/or $-NR^6$, where n and $R^6$ possess the meanings indicated in Claim 1.

11. Compounds according to one of Claims 8 to 10, characterised in that A in formula I is substituted by one, two or more radicals $-CO_2R^5$, where $R^5$ has the meaning indicated in Claim 1.

12. Process for the preparation of polysulphuric acid esters of bis-aldonamides and their derivatives of the formula I according to Claim 1, characterised in that bis-aldonamides of the general formula IX,

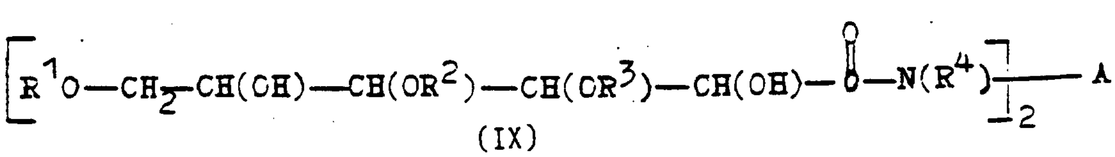

(IX)

in which $R^1$, $R^2$, $R^3$, $R^4$ and A have the meanings indicated in Claim 1, where, however, X in the formulae II to VII stands for a hydrogen atom, are reacted with a sulphating agent in an aprotic solvent

and the products thus obtained are optionally converted into the corresponding salts using an inorganic or organic base.

**13.** Process according to Claim 12, characterised in that in each case, without isolation of intermediates, aldonic acids of the general formula VIII,

$R^1O—CH_2—CH(OH)—CH(OR^2)—CH(OR^3)—CH(OH)—CO_2H$ (VIII)

in which $R^1$, $R^2$ and $R^3$ have the meanings indicated in Claim 1, where, however, X in the formulae II to VII stands for a hydrogen atom, are converted into the bis-aldonamides of the general formula IX and reacted to give their polysulphuric acid esters.

**14.** Medicaments, containing one or more compounds of the general formula I, according to one of Claims 1 to 11, together with customary excipients and auxiliaries.

**Claims for the following Contracting State : ES**

**1.** Process for the preparation of polysulphuric acid esters of bis-aldonamides and their derivatives of the general formula I

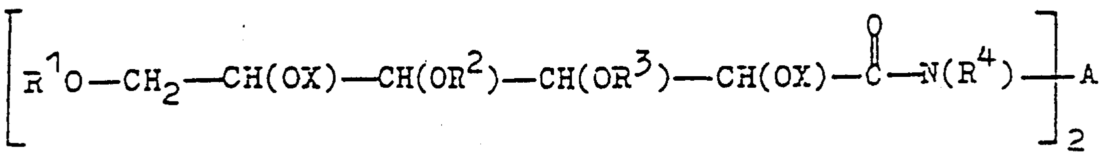

(I)

in which either
all radicals $R^1$, $R^2$ and $R^3$ independently of one another stand for X, or
two of the radicals $R^1$, $R^2$ and $R^3$ stand for X, and the third stands for a radical of the formulae II-VII

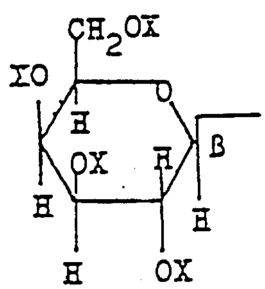

(II)

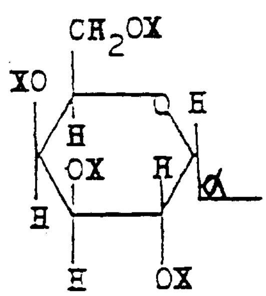

(III)

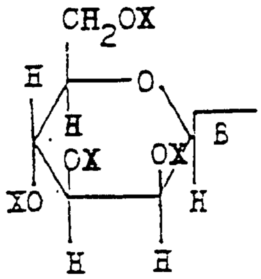

(IV)

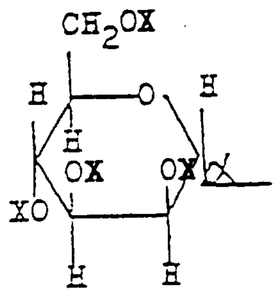

(V)

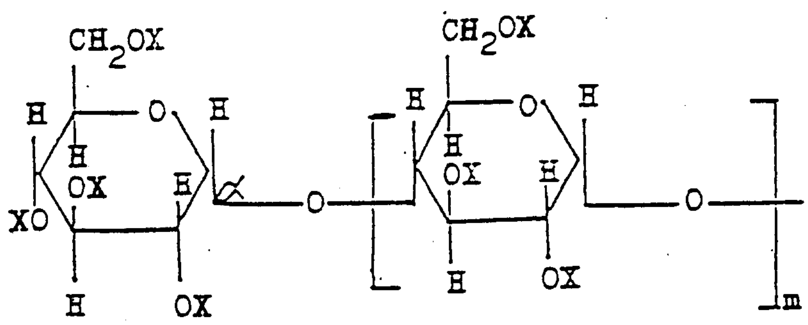

(VI)

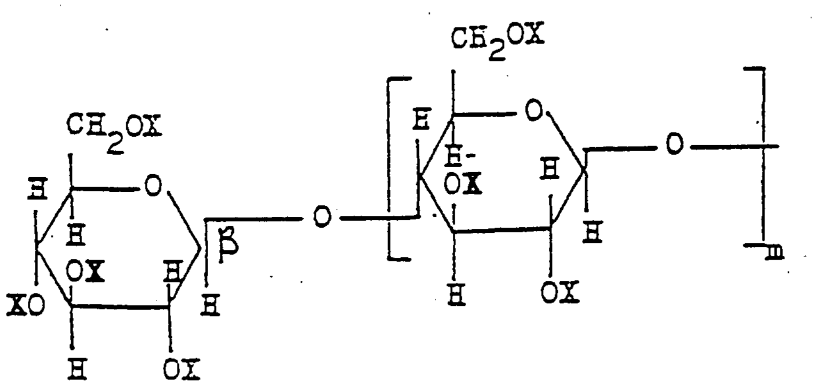

(VII)

X in the formulae I to VII simultaneously or independently of one another denotes a hydrogen atom or the group $-SO_3H$, where at leat one X stands for the group $-SO_3H$,

m stands for 0, 1, 2, 3, 4, 5 or 6,

A in formula I stands for a straight-chain or branched, saturated alkylene radical having 2 to 22 carbon atoms which is optionally substituted by one or more radicals $-CO_2R^5$, and this alkylene radical is optionally interrupted by up to 5 -O-, -S-, -S-S-, $-S(O)_n-$,

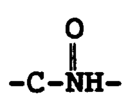

or/and $-NR^6$ groups or cycloalkylene or arylene radicals, or A stands for a single bond or the radical

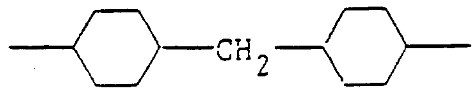

| | |
|---|---|
| n | is 1 or 2, |
| $R^4$, $R^5$ and $R^6$ | simultaneously or independently of one another denote a hydrogen atom or a $C_1$-$C_6$-alkyl radical, |

and their salts with inorganic or organic bases, characterised in that bis-aldonamides of the general formula IX,

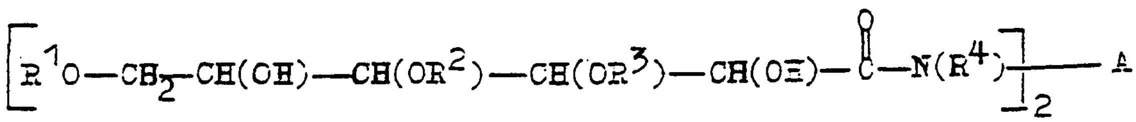

(IX)

in which $R^1$, $R^2$, $R^3$, $R^4$ and A have the meanings indicated above, where, however, X in the formulae II to VII stands for a hydrogen atom, are reacted with a sulphating agent in an aprotic solvent and the products thus obtained are optionally converted into the corresponding salts using an inorganic or organic base.

2. Process according to Claim 1, characterised in that in each case, without isolation of intermediates, aldonic acids of the general formula VIII,

$R^1O—CH_2—CH(OH)—CH(OR^2)—CH(OR^3)—CH(OH)—CO_2H$ (VIII)

in which $R^1$, $R^2$ and $R^3$ have the meanings indicated in Claim 1, where, however, X in the formulae II to VII stands for a hydrogen atom, are converted into the bis-aldonamides of the general formula IX and reacted to give their polysulphuric acid esters.

3. Process according to Claim 1 or 2, characterised in that each X in the formulae I to VII stands for the group $-SO_3H$.

4. Process according to Claim 1 or 2, characterised in that in the formulae I to VII at least half the X groups present represent an $-SO_3H$ group.

5. Process according to Claim 1 or 2, characterised in that $R^2$ and $R^3$ stand for X.

6. Process according to Claim 5, characterised in that $R^1$ denotes the radical III or the radical VI having m = 0.

7. Process according to Claim 1 or 2, characterised in that $R^1$ and $R^3$ stand for X.

8. Process according to Claim 7, characterised in that $R^2$ denotes the radical II or the radical VI having m = 0 or the radical VII having m = 0.

9. Process according to one of Claims 5 to 8, characterised in that A in formula I denotes a polymethylene radical $-(CH_2)_p-$ having p = 2 to 22.

10. Process according to one of Claims 5 to 8, characterised in that A in formula I denotes a polymethylene radical $-(CH_2)_p-$ having p = 2 to 12.

11. Process according to one of Claims 5 to 8, characterised in that A in formula I represents a straight-chain alkylene radical having 2 to 22 carbon atoms, whose chain can be interrupted by the groups -O-,

-S-, -S-S-, -S(O)$_n$-,

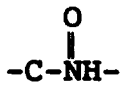

and/or -NR$^6$, where n and R$^6$ possess the meanings indicated in Claim 1.

**12.** Process according to one of Claims 9 to 11, characterised in that A in formula I is substituted by one, two or more radicals -CO$_2$R$^5$, where R$^5$ has the meaning indicated in Claim 1.

**Claims for the following Contracting State : GR**

**1.** Process for the preparation of polysulphuric acid esters of bis-aldonamides and their derivatives of the general formula I

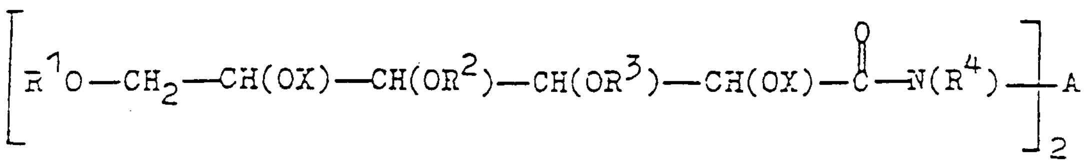 (I)

in which either
all radicals R$^1$, R$^2$ and R$^3$ independently of one another stand for X, or
two of the radicals R$^1$, R$^2$ and R$^3$ stand for X, and the third stands for a radical of the formulae II-VII

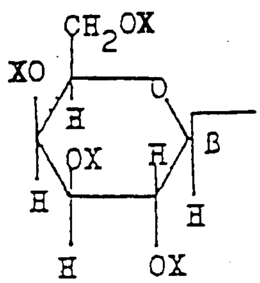

(II)

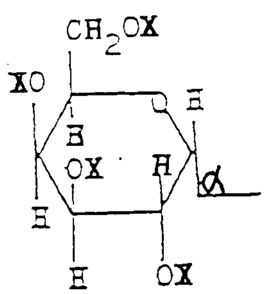

(III)

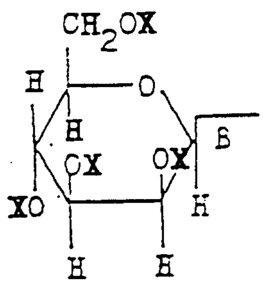

(IV)

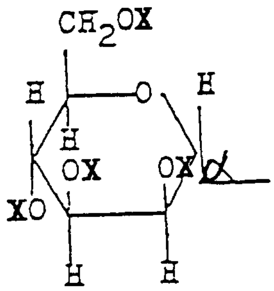

(V)

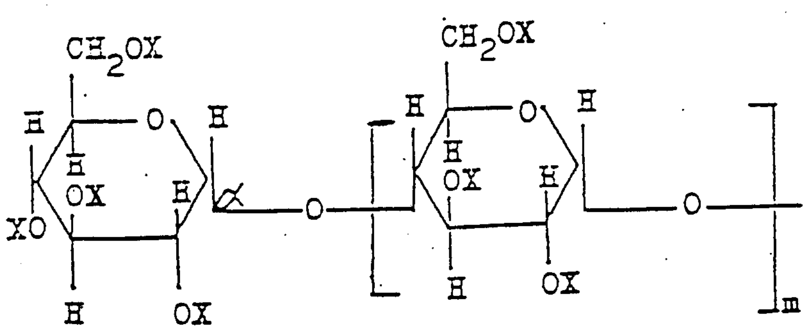

(VI)

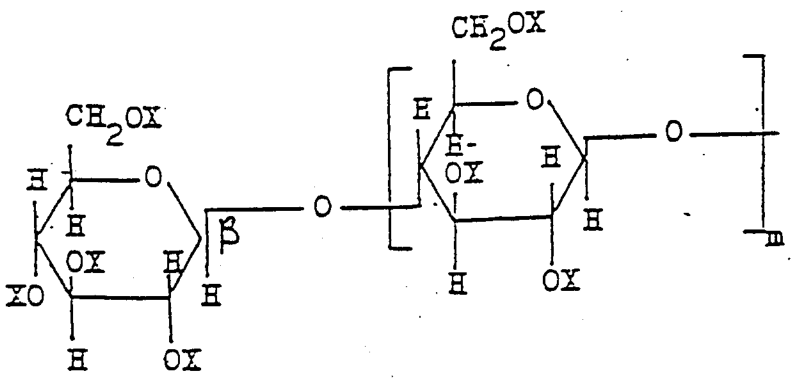

(VII)

X in the formulae I to VII simultaneously or independently of one another denotes a hydrogen atom or the group $-SO_3H$, where at leat one X stands for the group $-SO_3H$,
m stands for 0, 1, 2, 3, 4, 5 or 6,
A in formula I stands for a straight-chain or branched, saturated alkylene radical having 2 to 22 carbon atoms which is optionally substituted by one or more radicals $-CO_2R^5$, and this alkylene radical is optionally interrupted by up to 5 -O-, -S-, -S-S-, $-S(O)_n-$,

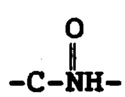

or/and $-NR^6$ groups or cycloalkylene or arylene radicals, or A stands for a single bond or the radical

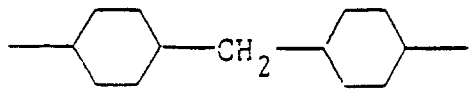

| | |
|---|---|
| n | is 1 or 2, |
| $R^4$, $R^5$ and $R^6$ | simultaneously or independently of one another denote a hydrogen atom or a $C_1$-$C_6$-alkyl radical, |

and their salts with inorganic or organic bases, characterised in that bis-aldonamides of the general formula IX,

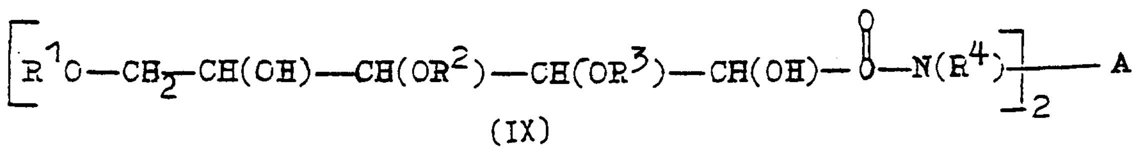

(IX)

in which $R^1$, $R^2$, $R^3$, $R^4$ and A have the meanings indicated above, where, however, X in the formulae II to VII stands for a hydrogen atom, are reacted with a sulphating agent in an aprotic solvent and the products thus obtained are optionally converted into the corresponding salts using an inorganic or organic base.

2. Process according to Claim 1, characterised in that in each case, without isolation of intermediates, aldonic acids of the general formula VIII,

$$R^1O{-}CH_2{-}CH(OH){-}CH(OR^2){-}CH(OR^3){-}CH(OH){-}CO_2H \quad (VIII)$$

in which $R^1$, $R^2$ and $R^3$ have the meanings indicated in Claim 1, where, however, X in the formulae II to VII stands for a hydrogen atom, are converted into the bis-aldonamides of the general formula IX and reacted to give their polysulphuric acid esters.

3. Process according to Claim 1 or 2, characterised in that each X in the formulae I to VII stands for the group $-SO_3H$.

4. Process according to Claim 1 or 2, characterised in that in the formulae I to VII at least half the X groups present represent an $-SO_3H$ group.

5. Process according to Claim 1 or 2, characterised in that $R^2$ and $R^3$ stand for X.

6. Process according to Claim 5, characterised in that $R^1$ denotes the radical III or the radical VI having m = 0.

7. Process according to Claim 1 or 2, characterised in that $R^1$ and $R^3$ stand for X.

8. Process according to Claim 7, characterised in that $R^2$ denotes the radical II or the radical VI having m = 0 or the radical VII having m = 0.

9. Process according to one of Claims 5 to 8, characterised in that A in formula I denotes a polymethylene radical $-(CH_2)_p-$ having p = 2 to 22.

10. Process according to one of Claims 5 to 8, characterised in that A in formula I denotes a polymethylene radical $-(CH_2)_p-$ having p = 2 to 12.

11. Process according to one of Claims 5 to 8, characterised in that A in formula I represents a straight-chain alkylene radical having 2 to 22 carbon atoms, whose chain can be interrupted by the groups -O-, -S-, -S-S-, $-S(O)_n-$,

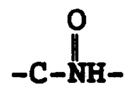

and/or $-NR^6$, where n and $R^6$ possess the meanings indicated in Claim 1.

**12.** Process according to one of Claims 9 to 11, characterised in that A in formula I is substituted by one, two or more radicals $-CO_2R^5$, where $R^5$ has the meaning indicated in Claim 1.

**13.** Use of polysulphuric acid esters of bis-aldonamides and their derivatives of the formula I, as defined in Claim 1, and of their salts with inorganic or organic bases to prepare medicaments.

**Revendications**

**Revendications pour les Etats contractants suivants : AT, BE, CH, LI, DE, FR, GB, IT, LU, NL, SE**

**1.** Esters polysulfuriques de bis-amides d'acides aldoniques et de leurs dérivés de formule générale I

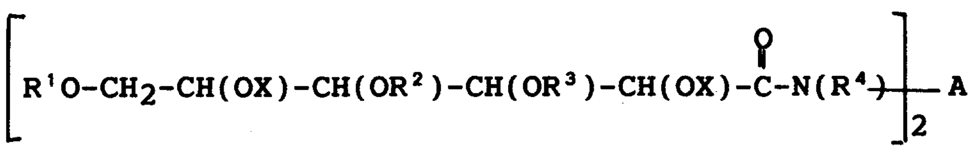 (I)

dans laquelle, soit

les restes $R^1$, $R^2$ et $R^3$, indépendamment l'un de l'autre, représentent tous X, soit

deux des restes $R^1$, $R^2$ et $R^3$ représentent X et le troisième représente un reste de l'une des formules II-VII,

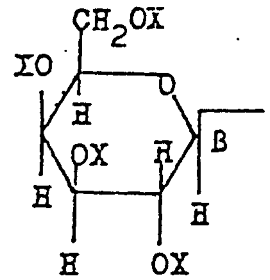

(II)

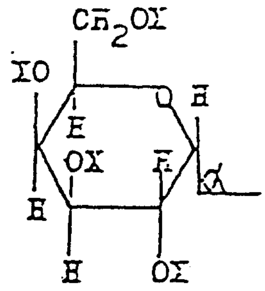

(III)

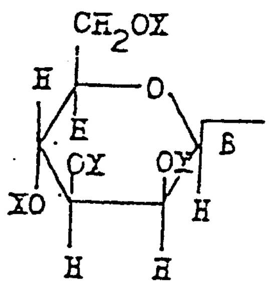

(IV)

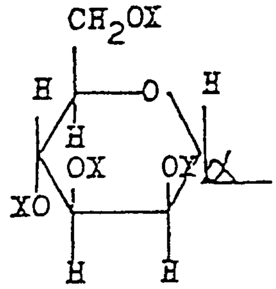

(V)

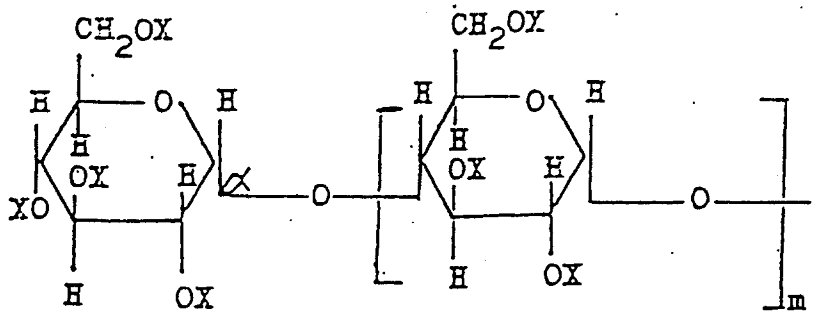

(VI)

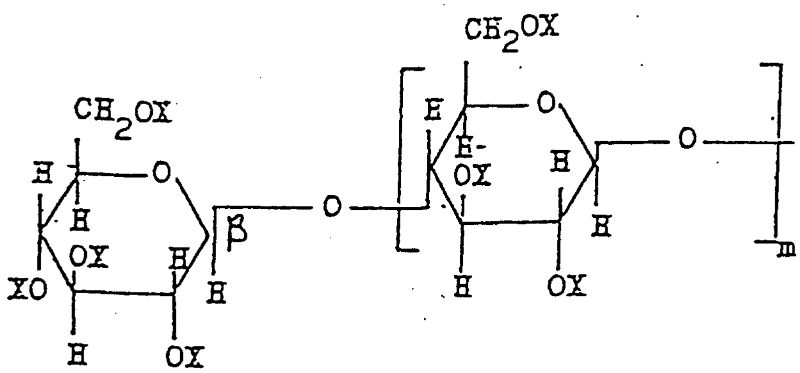

(VII)

les X représentent, dans les formules I à VII, simultanément ou indépendamment l'un de l'autre, un atome d'hydrogène ou le groupe $-SO_3H$, au moins l'un des X étant un groupe $SO_3H$,

m signifie 0, 1, 2, 3, 4, 5 ou 6,

A dans la formule I représente un reste alkylène de 2 à 22 atomes de carbone saturé, linéaire ou ramifié, éventuellement substitué par un ou plusieurs restes $CO_2R^5$, et ce reste alkylène est éventuellement interrompu jusqu'à 5 fois par des groupes -O-, -S-, -S-S-, $-S(O)_n-$,

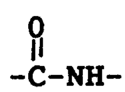

ou/et $-NR^6-$ ou par des restes cycloalkylène ou arylène, ou A représente une liaison simple ou le reste

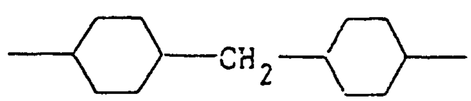

n est 1 ou 2,

$R^4$, $R^5$ et $R^6$ représentent simultanément ou indépendamment l'un de l'autre un atome d'hydrogène ou un reste alkyle en $C_1$-$C_6$,
et leurs sels avec des bases inorganiques ou organiques.

**2.** Composés selon la revendication 1, caractérisés en ce que, dans les formules I à VII, chacun des X représente le groupe $-SO_3H$.

**3.** Composés selon la revendication 1, caractérisés en ce que, dans les formules I à VII, au moins la moitié des groupes X présents représentent un groupe $-SO_3H$.

**4.** Composés selon la revendication 1, caractérisés en ce que $R^2$ et $R^3$ représentent X.

**5.** Composés selon la revendication 4, caractérisés en ce que $R^1$ représente le reste III ou le reste VI avec m = 0.

**6.** Composés selon la revendication 1, caractérisés en ce que $R^1$ et $R^3$ représentent X.

**7.** Composés selon la revendication 6, caractérisés en ce que $R^2$ représente le reste II ou le reste VI avec m = 0 ou le reste VII avec m = 0.

**8.** Composés selon l'une des revendications 4 à 7, caractérisés en ce que, dans la formule I, A représente un reste polyméthylène $-(CH_2)_p-$ avec p = 2 à 22.

**9.** Composés selon l'une des revendications 4 à 7, caractérisés en ce que, dans la formule I, A représente un reste polyméthylène $-(CH_2)_p-$ avec p = 2 à 12.

**10.** Composés selon l'une des revendications 4 à 7, caractérisés en ce que, dans la formule I, A représente un reste alkylène linéaire de 2 à 22 atomes de carbone dont la chaîne peut être interrompue par les groupes -O-, -S-, -S-S-, $-S(O)_n-$,

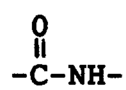

ou/et $-NR^6-$, n et $R^6$ ayant les significations données dans la revendication 1.

**11.** Composés selon l'une des revendications 8 à 10, caractérisés en ce que, dans la formule I, A est substitué par un, deux ou plusieurs restes $-CO_2R^5$, $R^5$ ayant la signification donnée dans la revendication 1.

**12.** Procédé de préparation d'esters polysulfuriques de bis-amides d'acides aldoniques et de leurs dérivés de formule I selon la revendication 1, caractérisé en ce que l'on fait réagir des bis-amides d'acides aldoniques de formule générale IX

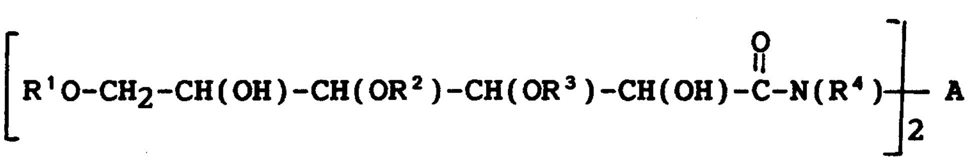

**(IX)**

dans laquelle $R^1$, $R^2$, $R^3$, $R^4$ et A ont les significations indiquées dans la revendication 1, X représentant cependant un atome d'hydrogène dans les formules II à VII, avec un agent de sulfatation dans un solvant aprotique, et en ce qu'on transforme éventuellement les produits ainsi obtenus avec une base inorganique ou organique en les sels correspondants.

**13.** Procédé selon la revendication 12, caractérisé en ce que, sans isoler les produits intermédiaires, on transforme des acides aldoniques de formule générale VIII

$R^1O\text{-}CH_2\text{-}CH(OH)\text{-}CH(OR^2)\text{-}CH(OR^3)\text{-}CH(OH)\text{-}CO_2H$ (VIII)

dans laquelle $R^1$, $R^2$ et $R^3$ ont les significations données dans la revendication 1, X représentant toutefois un atome d'hydrogène dans les formules II à VII, en les bis-amides d'acides aldoniques de formule générale IX que l'on transforme en leurs esters polysulfuriques.

**14.** Médicaments contenant un ou plusieurs composés de formule générale I selon l'une des revendications 1 à 11 avec des supports et des adjuvants classiques.

**Revendications pour l'Etat contractant suivant : ES**

**1.** Procédé de préparation d'esters polysulfuriques de bis-amides d'acides aldoniques et de leurs dérivés de formule générale I

$$\left[R^1O\text{-}CH_2\text{-}CH(OX)\text{-}CH(OR^2)\text{-}CH(OR^3)\text{-}CH(OX)\text{-}\overset{\overset{O}{\|}}{C}\text{-}N(R^4)\right]_2 A \qquad (I)$$

dans laquelle, soit

les restes $R^1$, $R^2$ et $R^3$, indépendamment l'un de l'autre, représentent tous X, soit

deux des restes $R^1$, $R^2$ et $R^3$ représentent X et le troisième représente un reste de l'une des formules II-VII,

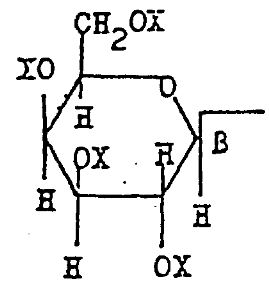

(II)

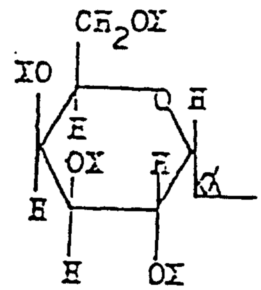

(III)

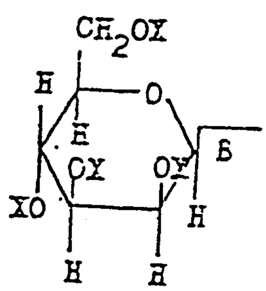

(IV)

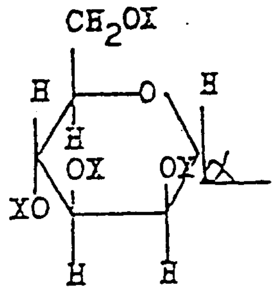

(V)

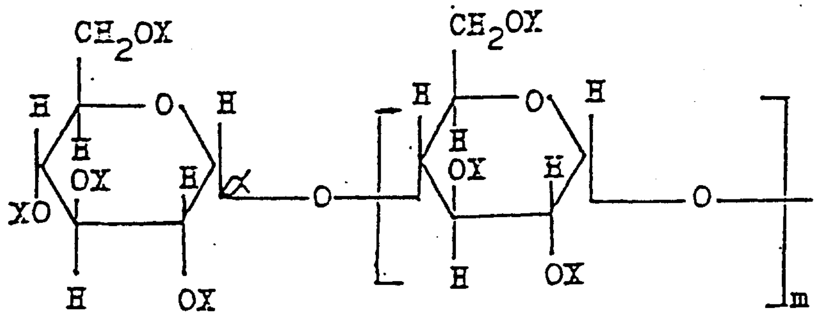

(VI)

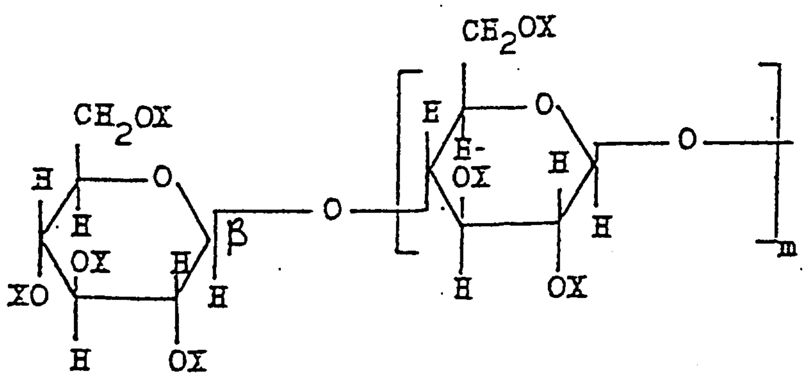

(VII)

les X représentent, dans les formules I à VII, simultanément ou indépendamment l'un de l'autre, un atome d'hydrogène ou le groupe $-SO_3H$, au moins l'un des X étant un groupe $SO_3H$,

m signifie 0, 1, 2, 3, 4, 5 ou 6,

A dans la formule I représente un reste alkylène de 2 à 22 atomes de carbone saturé, linéaire ou ramifié, éventuellement substitué par un ou plusieurs restes $CO_2R^5$, et ce reste alkylène est éventuellement interrompu jusqu'à 5 fois par des groupes -O-, -S-, -S-S-, $-S(O)_n-$,

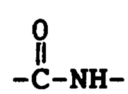

ou/et $-NR^6-$ ou par des restes cycloalkylène ou arylène, ou A représente une liaison simple ou le reste

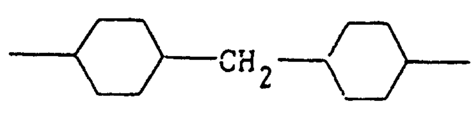

n est 1 ou 2,

$R^4$, $R^5$ et $R^6$ représentent simultanément ou indépendamment l'un de l'autre un atome d'hydrogène ou un reste alkyle en $C_1$-$C_6$,

et de leurs sels avec des bases inorganiques ou organiques, caractérisé en ce que l'on fait réagir des bis-amides d'acides aldoniques de formule générale IX

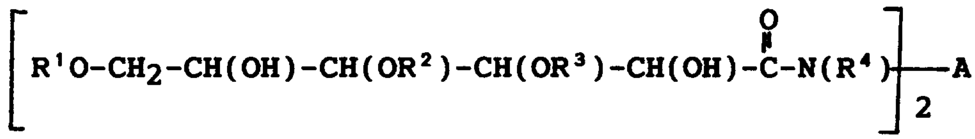

(IX)

dans laquelle $R^1$, $R^2$, $R^3$, $R^4$ et A ont les significations indiquées dans la revendication 1, X représentant cependant un atome d''hydrogène dans les formules II à VII, avec un agent de sulfatation dans un solvant aprotique, et en ce qu'on transforme éventuellement les produits ainsi obtenus avec une base inorganique ou organique en les sels correspondants.

**2.** Procédé selon la revendication 1, caractérisé en ce que, sans isoler les produits intermédiaires, on transforme des acides aldoniques de formule générale VIII

$R^1O\text{-}CH_2\text{-}CH(OH)\text{-}CH(OR^2)\text{-}CH(OR^3)\text{-}CH(OH)\text{-}CO_2H$ (VIII)

dans laquelle $R^1$, $R^2$ et $R^3$ ont les significations données dans la revendication 1, X représentant toutefois un atome d'hydrogène dans les formules II à VII, en les bis-amides d'acides aldoniques de formule générale IX que l'on transforme en leurs esters polysulfuriques.

**3.** Procédé selon la revendication 1 ou 2, caractérisé en ce que, dans les formules I à VII, chacun des X représente le groupe $-SO_3H$.

**4.** Procédé selon la revendication 1 ou 2, caractérisé en ce que, dans les formules I à VII, au moins la moitié des groupes X présents représentent un groupe $-SO_3H$.

**5.** Procédé selon la revendication 1 ou 2, caractérisé en ce que $R^2$ et $R^3$ représentent X.

**6.** Procédé selon la revendication 5, caractérisé en ce que $R^1$ représente le reste III ou le reste VI avec m = 0.

**7.** Procédé selon la revendication 1 ou 2, caractérisé en ce que $R^1$ et $R^3$ représentent X.

**8.** Procédé selon la revendication 7, caractérisé en ce que $R^2$ représente le reste II ou le reste VI avec m = 0 ou le reste VII avec m = 0.

**9.** Procédé selon l'une des revendications 5 à 8, caractérisé en ce que, dans la formule I, A représente un reste polyméthylène $-(CH_2)_p-$ avec p = 2 à 22.

**10.** Procédé selon l'une des revendications 5 à 8, caractérisé en ce que, dans la formule I, A représente un reste polyméthylène $-(CH_2)_p-$ avec p = 2 à 12.

**11.** Procédé selon l'une des revendications 5 à 8, caractérisé en ce que, dans la formule I, A représente un reste alkylène linéaire de 2 à 22 atomes de carbone dont la chaîne peut être interrompue par les groupes -O-, -S-, -S-S-, $-S(O)_n-$,

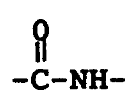

ou/et $-NR^6-$, n et $R^6$ ayant les significations données dans la revendication 1.

**12.** Procédé selon l'une des revendications 9 à 11, caractérisé en ce que, dans la formule I, A est substitué par un, deux ou plusieurs restes $-CO_2R^5$, $R^5$ ayant la signification donnée dans la revendication 1.

**Revendications pour l'Etat contractant suivant : GR**

**1.** Procédé de préparation d'esters polysulfuriques de bis-amides d'acides aldoniques et de leurs dérivés de formule générale I

$$\left[R^1O-CH_2-CH(OX)-CH(OR^2)-CH(OR^3)-CH(OX)-\overset{O}{\overset{\|}{C}}-N(R^4)\right]_2-A \qquad (I)$$

dans laquelle, soit

les restes $R^1$, $R^2$ et $R^3$, indépendamment l'un de l'autre, représentent tous X, soit

deux des restes $R^1$, $R^2$ et $R^3$ représentent X et le troisième représente un reste de l'une des formules II-VII,

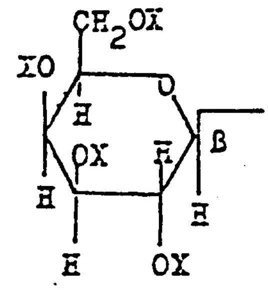

(II)

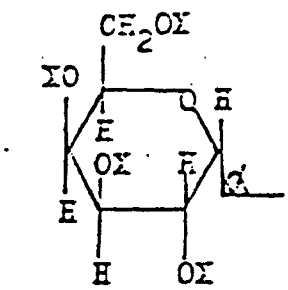

(III)

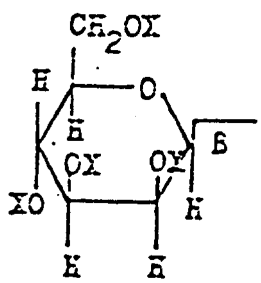

(IV)

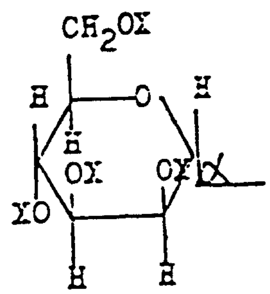

(V)

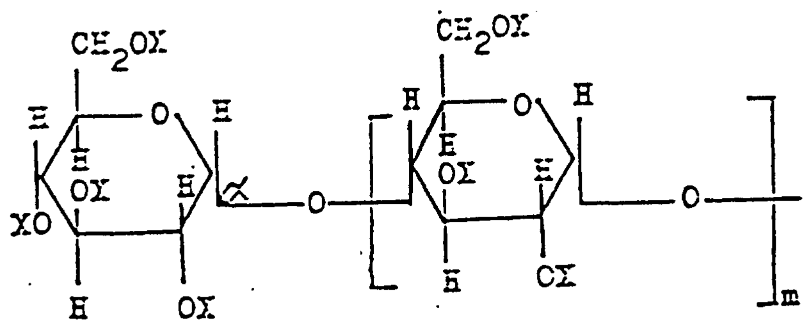

(VI)

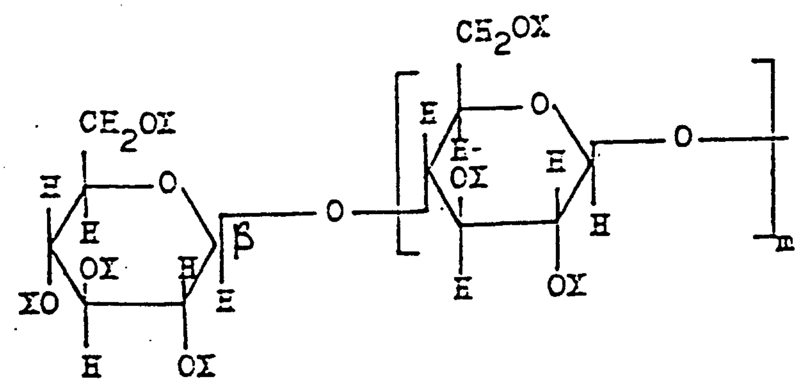

(VII)

les X représentent, dans les formules I à VII, simultanément ou indépendamment l'un de l'autre, un atome d'hydrogène ou le groupe $-SO_3H$, au moins l'un des X étant un groupe $SO_3H$,

m signifie 0, 1, 2, 3, 4, 5 ou 6,

A dans la formule I représente un reste alkylène de 2 à 22 atomes de carbone saturé, linéaire ou ramifié, éventuellement substitué par un ou plusieurs restes $CO_2R^5$, et ce reste alkylène est éventuellement interrompu jusqu'à 5 fois par des groupes -O-, -S-, -S-S-, $-S(O)_n-$,

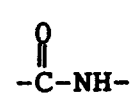

ou/et $-NR^6-$ ou par des restes cycloalkylène ou arylène, ou A représente une liaison simple ou le reste

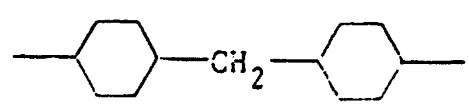

n est 1 ou 2,

$R^4$, $R^5$ et $R^6$ représentent simultanément ou indépendamment l'un de l'autre un atome d'hydrogène ou un reste alkyle en $C_1$-$C_6$,

et de leurs sels avec des bases inorganiques ou organiques, caractérisé en ce que l'on fait réagir des

bis-amides d'acides aldoniques de formule générale IX

$$\left[R^1O\text{-}CH_2\text{-}CH(OH)\text{-}CH(OR^2)\text{-}CH(OR^3)\text{-}CH(OH)\text{-}\overset{\overset{\displaystyle O}{\|}}{C}\text{-}N(R^4)\right]_2\!\!\text{—}A$$

(IX)

dans laquelle $R^1$, $R^2$, $R^3$, $R^4$ et A ont les significations indiquées dans la revendication 1, X représentant cependant un atome d'hydrogène dans les formules II à VII, avec un agent de sulfatation dans un solvant aprotique, et en ce qu'on transforme éventuellement les produits ainsi obtenus avec une base inorganique ou organique en les sels correspondants.

**2.** Procédé selon la revendication 1, caractérisé en ce que, sans isoler les produits intermédiaires, on transforme des acides aldoniques de formule générale VIII

$R^1O\text{-}CH_2\text{-}CH(OH)\text{-}CH(OR^2)\text{-}CH(OR^3)\text{-}CH(OH)\text{-}CO_2H$ (VIII)

dans laquelle $R^1$, $R^2$ et $R^3$ ont les significations données dans la revendication 1, X représentant toutefois un atome d'hydrogène dans les formules II à VII, en les bis-amides d'acides aldoniques de formule générale IX que l'on transforme en leurs esters polysulfuriques.

**3.** Procédé selon la revendication 1 ou 2, caractérisé en ce que, dans les formules I à VII, chacun des X représente le groupe $-SO_3H$.

**4.** Procédé selon la revendication 1 ou 2, caractérisé en ce que, dans les formules I à VII, au moins la moitié des groupes X présents représentent un groupe $-SO_3H$.

**5.** Procédé selon la revendication 1 ou 2, caractérisé en ce que $R^2$ et $R^3$ représentent X.

**6.** Procédé selon la revendication 5, caractérisé en ce que $R^1$ représente le reste III ou le reste VI avec m = 0.

**7.** Procédé selon la revendication 1 ou 2, caractérisé en ce que $R^1$ et $R^3$ représentent X.

**8.** Procédé selon la revendication 7, caractérisé en ce que $R^2$ représente le reste II ou le reste VI avec m = 0 ou le reste VII avec m = 0.

**9.** Procédé selon l'une des revendications 5 à 8, caractérisé en ce que, dans la formule I, A représente un reste polyméthylène $-(CH_2)_p-$ avec p = 2 à 22.

**10.** Procédé selon l'une des revendications 5 à 8, caractérisé en ce que, dans la formule I, A représente un reste polyméthylène $-(CH_2)_p-$ avec p = 1 à 22.

**11.** Procédé selon l'une des revendications 5 à 8, caractérisé en ce que, dans la formule I, A représente un reste alkylène linéaire de 2 à 22 atomes de carbone dont la chaîne peut être interrompue par les groupes -O-, -S-, -S-S-, $-S(O)_n-$,

$$-\overset{\overset{\displaystyle O}{\|}}{C}-NH-$$

ou/et $-NR^6-$, n et $R^6$ ayant les significations données dans la revendication 1.

**12.** Procédé selon l'une des revendications 9 à 11, caractérisé en ce que, dans la formule I, A est substitué par un, deux ou plusieurs restes $-CO_2R^5$, $R^5$ ayant la signification donnée dans la revendication 1.

**13.** Utilisation d'esters polysulfuriques de bis-amides d'acides aldoniques et de leurs dérivés de formule I, tels que définis dans la revendication 1, et de leurs sels avec des bases inorganiques ou organiques, pour la préparation de médicaments.